(19) Europäisches Patentamt
European Patent Office
Office européen des brevets

(11) **EP 4 288 133 B1**

(12) **EUROPÄISCHE PATENTSCHRIFT**

(45) Veröffentlichungstag und Bekanntmachung des Hinweises auf die Patenterteilung:
**30.04.2025 Patentblatt 2025/18**

(21) Anmeldenummer: **22709611.2**

(22) Anmeldetag: **07.02.2022**

(51) Internationale Patentklassifikation (IPC):
*A61M 16/00* *(2006.01)*

(52) Gemeinsame Patentklassifikation (CPC):
**A61M 16/024;** A61M 16/04; A61M 16/0816;
A61M 16/0833; A61M 16/0858; A61M 16/0875;
A61M 16/0883; A61M 16/109; A61M 16/16;
A61M 2016/0027; A61M 2016/0036;
A61M 2205/502; A61M 2209/045; A61M 2209/084

(86) Internationale Anmeldenummer:
**PCT/EP2022/052837**

(87) Internationale Veröffentlichungsnummer:
**WO 2022/167638 (11.08.2022 Gazette 2022/32)**

(54) **VERFAHREN UND BEATMUNGSVORRICHTUNG ZUR BESTIMMUNG EINES ATEMTRAKTINHALTS AN ATEMGAS WÄHREND EINER KÜNSTLICHEN BEATMUNG**

METHOD AND VENTILATION APPARATUS FOR DETERMINING A RESPIRATORY-GAS CONTENT IN A RESPIRATORY TRACT DURING ARTIFICIAL VENTILATION

PROCÉDÉ ET APPAREIL DE VENTILATION PERMETTANT DE DÉTERMINER UNE TENEUR EN GAZ RESPIRATOIRE DANS LES VOIES RESPIRATOIRES PENDANT UNE VENTILATION ARTIFICIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.02.2021 DE 102021102886**

(43) Veröffentlichungstag der Anmeldung:
**13.12.2023 Patentblatt 2023/50**

(73) Patentinhaber: **Hamilton Medical AG**
**7402 Bonaduz (CH)**

(72) Erfinder:
• **BEDA, Alessandro**
**7403 Rhäzüns (CH)**

• **WADEHN, Federico Alexander**
**7402 Bonaduz (CH)**
• **MEYER, Johannes**
**7000 Chur (CH)**
• **NOVOTNI, Dominik**
**7000 Chur (CH)**

(74) Vertreter: **Ruttensperger Lachnit Trossin Gomoll Patent- und Rechtsanwälte PartG mbB Arnulfstraße 58 80335 München (DE)**

(56) Entgegenhaltungen:
**WO-A1-2018/089837 WO-A1-2019/094736 US-A1- 2012 137 249**

**Beschreibung**

Die vorliegende Erfindung betrifft ein Verfahren zur Bestimmung eines Atemtraktinhalts an Atemgas, welcher nach mehreren unter Beteiligung einer Beatmungsvorrichtung ausgeführten Atemhüben im Atemtrakt eines wenigstens teilweise künstlich beatmeten Patienten vorhanden ist, wobei die mehreren Atemhübe wenigstens eine frühere Atemhubmenge und wenigstens einen auf die frühere Atemhubmenge folgenden Folge-Atemhub aufweisen. Die frühere Atemhubmenge weist einen früheren Atemhub oder eine Mehrzahl von aufeinanderfolgenden früheren Atemhüben auf. Das Verfahren umfasst ein quantitatives Erfassen von inspiratorischen und exspiratorischen Atemgasflüssen durch wenigstens einen Atemgas-Flusssensor und es umfasst eine Summation von erfassten inspiratorischen und exspiratorischen Flusswerten zu dem Atemgas-Atemtraktinhalt.

Die Erfindung betrifft außerdem eine Beatmungsvorrichtung zur wenigstens teilweise künstlichen Beatmung eines Patienten, welche zur Ausführung des vorliegend beschriebenen Verfahrens ausgebildet ist.

Ein solches Verfahren und eine dieses Verfahren nutzende Beatmungsvorrichtung sind aus der WO 2019/094736 A1 bekannt. Die WO 2019/094736 A1 lehrt, auf ein weiter unten erläutertes Beatmungsphänomen des "double triggering" dann zu schließen, wenn die Exspirationsdauer in einem vorbestimmten Verhältnis zur Inspirationsdauer steht bzw. auf ein solches vorbestimmtes Verhältnis gestützte Schwellenwerte über- bzw. unterschreitet.

Die WO 2019/094736 A1 lehrt außerdem, auf ein dem "double triggering" ähnliches Beatmungsphänomen des "Air Trapping" dann zu schließen, wenn das Exspirationsvolumen geringer als das Inspirationsvolumen ist, insbesondere wenn der Unterschied zwischen Exspirationsvolumen und Inspirationsvolumen einen vorbestimmten Schwellenwert überschreitet. Die Folge einer Erkennung eines "Air Trapping" ist gemäß der WO 2019/094736 A1 die Kennzeichnung des betreffenden Atemhubs als Air-Trapping-Atemhub.

Grundsätzlich werden Atemgasvolumina an Beatmungsvorrichtungen durch Integration, aufgrund der Verwendung digitaler Datenverarbeitung in der Regel numerischer Integration, der erfassten Atemgasflüsse über die interessierende Zeit ermittelt. So wird an Beatmungsvorrichtungen die verabreichte inspiratorische Atemgasmenge durch Integration der erfassten inspiratorischen Atemgasflüsse, also der zum Patienten hin strömenden Atemgasflüsse, vom Beginn des Inspirationsvorgangs bis zu dessen Ende ermittelt. Ebenso kann die vom Patienten abgegebene exspiratorische Atemgasmenge durch Integration der erfassten exspiratorischen Atemgasflüsse über die Dauer des Exspirationsvorgangs, also vom Beginn des Exspirationsvorgangs bis zu dessen Ende, ermittelt werden.

Die Kenntnis von beispielsweise inspiratorischen Atemgasvolumina kann für die Steuerung einer Beatmungsvorrichtung wichtig sein, um zu ermitteln, ob einem Patienten das vom behandelnden Personal bestimmte Tidalvolumen (das ist bei volumenbezogener Beatmungssteuerung die pro Atemhub zu verabreichende Atemgasmenge) verabreicht wurde. Ist dies der Fall, steuert beispielsweise eine in einem volumenbezogen gesteuerten Beatmungsmodus betriebene Beatmungsvorrichtung von Inspiration auf Exspiration um.

Häufig werden Atemgasflüsse kontinuierlich erfasst und integriert, um das jeweils zu einem Zeitpunkt im Atemtrakt des Patienten vorhandene oder verbliebene Atemgas zu kennen. Dabei werden inspiratorische und exspiratorische Atemgasflüsse aufgrund ihrer entgegengesetzten Richtung, einmal zum Patienten hin und einmal vom Patienten weg, durch unterschiedliche mathematische Vorzeichen unterschieden. Bei der Integration der Atemgasströme wird so während eines Inspirationsvorgangs zunächst eine inspiratorische Atemgasmenge im Patienten betragsmäßig akkumuliert und anschließend während eines nachfolgenden Exspirationsvorgangs wieder betragsmäßig verringert.

Idealerweise strömt während der Exspirationsphase die gleiche Atemgasmenge vom Patienten weg, die zuvor in einer Inspirationsphase zum Patienten hin geströmt ist. Allerdings kann es vorkommen, dass Flusssensoren in Beatmungsvorrichtungen die Atemgasströmungen in entgegengesetzten Strömungsrichtungen mit unterschiedlicher Genauigkeit erfassen, sei es aufgrund eines Kalibrationsmangels oder sei es aufgrund von Fertigungsungenauigkeiten, welche sich in richtungsmäßig asymmetrischer Erfassungsgenauigkeit manifestieren können. Ein Kalibrationsmangel kann beispielsweise durch Belastung eines Flusssensors mit Feuchtigkeit aus dem Atemgas entstehen, die sich im Bereich des Flusssensors niederschlägt. Durch zunehmende oder sich ändernde Feuchtigkeitsbelastung weicht der Flusssensor zunehmend oder in sich änderndem Maße von seiner Kalibration ab. Die Feuchtigkeit kann sich stromaufwärts und stromabwärts des Flusssensors, insbesondere eines Differenzdruck-Flusssensors, unterschiedlich stark niederschlagen und so ein hinsichtlich der Strömungsrichtung des Atemgases asymmetrisches Erfassungsverhalten begründen.

Die Folge ist dann eine über mehrere Atemhübe beobachtbare Drift des das Atemvolumen repräsentierenden Sensorsignals. Aufgrund dieser Drift weicht das Sensorsignal mit fortschreitender Zeit betragsmäßig zunehmend von einem theoretischen fehlerfreien Sensorsignal ab.

Um eine solche Drift zu vermeiden sind einige Beatmungsvorrichtungen des Standes der Technik dazu ausgebildet, für jeden Atemhub den Atemtraktinhalt des Patienten an Atemgas (Atemgas-Atemtraktinhalt) unabhängig vom Ergebnis der Erfassung des Atemtraktinhalts für den unmittelbar vorhergehenden Atemhub gesondert zu bestimmen. Dies entspricht bei einer kontinuierlichen Erfassung des Atemgas-Atemtraktinhalts einem Rücksetzen des erfassten Atemtraktinhalts am Ende eines früheren Atemhubs auf null, sodass die Erfassung des Atemtraktinhalts für den Folge-Atemhub bei null beginnt.

Damit kann zwar die beschriebene Drift vermieden werden, jedoch besteht die Gefahr, dass ein solches regelmäßiges Rücksetzen der summarischen Erfassung der inspiratorischen und exspiratorischen Atemgasvolumina andere für die Bewertung eines Beatmungsvorgangs relevante Ereignisse verdeckt, wie beispielsweise das sogenannte "breath stacking", etwa ausgelöst durch "double triggering" oder durch eine kurze Exspiration, was im Deutschen als "Atemstapelung" verstanden werden kann. Bei diesem Ereignis findet zwischen zwei ausgelösten Inspirationsvorgängen keine oder nur eine unvollständige Exspiration statt, sodass im Folge-Atemhub nach der unvollständigen Exspiration auf die im Atemtrakt des Patienten als Atemtraktinhalt verbliebene signifikante Atemgas-Restmenge erneut das an der Beatmungsvorrichtung vorgegebene Tidalvolumen verabreicht wird. Da der im Atemtrakt des Patienten am Ende des unvollständigen Exspirationsvorgangs des früheren Atemhubs verbliebene Atemtraktinhalt wegen des Rücksetzens auf null bei der Erfassung der Atemgasvolumina nicht erkannt wird, wird mit der Verabreichung des Tidalvolumens im Folge-Atemhub im Atemtrakt des Patienten ein unerwünscht hoher Atemgasdruck und ein unerwünscht hohes Atemgasvolumen erzeugt, was für den Patienten im geringsten Fall unangenehm ist, jedoch ein unerwünschtes medizinisches Risiko bedeutet.

Aus der US 8757152 B1 sind verschiedene Verfahren bekannt, auf Grundlage von Beatmungsparametern eines aktuellen Beatmungsvorgangs auf ein Auftreten einer Doppelauslösung eines Inspirationsvorgangs ("double triggering") zu schließen. Diese Verfahren haben jedoch keinen Einfluss auf die Bestimmung des Atemgas-Atemtraktinhalts in einem auf einen früheren Atemhub folgenden Folge-Atemhub.

Es ist daher Aufgabe der vorliegenden Erfindung, das eingangs genannte Verfahren zur Bestimmung eines Atemgas-Atemtraktinhalts und eine zur Ausführung des Verfahrens ausgebildete Beatmungsvorrichtung derart weiterzubilden, das eine Drift eines den Atemgas-Atemtraktinhalt eines Patienten repräsentierenden Sensorsignals ausreichend sicher vermieden werden kann, ohne dadurch eine medizinisch relevante Atemstapelung auszublenden.

Die vorliegende Erfindung löst diese Aufgabe an dem eingangs genannten Verfahren dadurch, dass ein Atemtraktinhalt-Anfangswert, mit welchem die Bestimmung des Atemgas-Atemtraktinhalts für den Folge-Atemhub beginnt, abhängig von einem Atemgasmengen-Unterschied zwischen einer während der früheren Atemhubmenge dem Patienten zugeführten inspiratorischen Atemgasmenge und einer während der früheren Atemhubmenge vom Patienten abgegebenen exspiratorischen Atemgasmenge auf einen näher bei null als bei einem den Atemgasmengen-Unterschied quantitativ angebenden Unterschiedswert gelegenen Rücksetz-Anfangswert oder auf einen näher beim Unterschiedswert als bei null gelegenen Kontinuitäts-Anfangswert gesetzt wird. Somit kann beispielsweise dann, wenn der Atemgasmengen-Unterschied betragsmäßig klein ist, etwa kleiner als ein Unterschied-Schwellenwert, der Rücksetz-Anfangswert als Atemtraktinhalt-Anfangswert gesetzt werden. Dies sollte der Regelfall einer künstlichen Beatmung sein, so dass für durch Verwendung des Rücksetz-Anfangswerts die beschriebene Drift verhindert werden kann. Außerdem kann dann, wenn der Atemgasmengen-Unterschied betragsmäßig groß ist, etwa größer als der Unterschied-Schwellenwert, der Kontinuitäts-Anfangswert als der Atemtraktinhalt-Anfangswert gesetzt werden. Dies ermöglicht es, außergewöhnliche Beatmungsereignisse zu bemerken, die sich in der vom Patienten aufgenommenen und abgegebenen Atemgasmenge abbilden, und dennoch eine unerwünschte Signaldrift zu vermeiden.

Somit können kleine betragsmäßige Abweichungen der inspiratorischen und der exspiratorischen Atemgasmenge voneinander nivelliert bzw. ausgeblendet werden, während für eine Berücksichtigung betragsmäßig ausreichend große Abweichungen als Anfangswert in die Bestimmung des Atemgas-Atemtraktinhalts für den Folge-Atemhub übertragen werden. Folglich wird bei der Bestimmung des Atemgas-Atemtraktinhalts für den Folge-Atemhub berücksichtigt, dass der Atemtrakt des Patienten zu Beginn des Folge-Atemhubs bereits teilweise mit Atemgas gefüllt ist.

Mit dem Begriff "Atemtrakt" sind die oberen Atemwege und die unteren Atemwege eines Patienten bezeichnet. Das gemeinhin als "Lunge" bezeichnete Körperorgan zählt zu den unteren Atemwegen.

Bevorzugt ist die Summation der inspiratorischen und der exspiratorischen Atemgasflüsse über die Mehrzahl von Atemhüben hinweg eine bilanzielle Summation, bei welcher entgegengesetzt gerichtete Atemgasflüsse mit unterschiedlichen mathematischen Vorzeichen berücksichtigt werden. Beispielsweise können alle dem Patienten zugeführten Atemgasflüsse positiv sein, während alle vom Patienten weg strömenden Atemgasflüsse negativ sind. Die fortwährende Summation von Atemgasflüssen mit unterschiedlichen Vorzeichen führt dazu, dass gleichgerichtete Atemgasflüsse ein und desselben Atemhubabschnitts, etwa eines Inspirationsvorgangs, zu einem betragsmäßig zunehmend größer werdenden inspiratorischen Atemgasvolumen summiert werden, und dass dieses Atemgasvolumen in einem anschließenden Exspirationsvorgang als einem weiteren Atemhubabschnitt mit innerhalb des Exspirationsvorgangs untereinander gleich, jedoch zum vorhergehenden Inspirationsvorgang entgegengesetzt gerichteten Atemgasflüssen durch die fortgesetzt summierten Atemgasflüsse mit entgegengesetzten Vorzeichen betragsmäßig wieder verringert wird. Zu jedem Zeitpunkt während eines Atemhubs gibt das so bestimmte Atemgasvolumen ausreichend genau den aktuellen Atemgas-Atemtraktinhalt des Patienten an. Beginnt die Bestimmung des Atemgas-Atemtraktinhalts zu Beginn des ersten von der Beatmungsvorrichtung unterstützten Atemhubs mit einem anfänglichen Atemgas-Atemtraktinhalt, so gibt das über die Mehrzahl von bisher abgelaufenen Atemgashüben ermittelte Atemgas-Atemtraktinhalt die Änderung gegenüber dem anfänglichen Atemgas-Atemtraktinhalt an. Da die Bestimmung eines in einem Atemtrakt eines Patienten vorhandenen Atemgas-Atemtraktinhalts außerhalb einer künstlichen Beatmung mit erheblichem Aufwand verbunden ist, ist der genannte anfängliche Atemgas-Atemtraktinhalt in der Regel null. Der mit dem vorliegend vorgeschlagenen Verfahren bestimmte Atemgas-Atemtraktinhalt ist daher bevorzugt ein Atemgas-Atemtraktinhalt zusätzlich zum in der Regel unbekannten anfänglichen Atemgas-Atemtraktinhalt. Der Atemgas-Atemtraktinhalt gibt also jenen Teil einer von der Beatmungsvorrichtung während einer Beatmungsbehandlung insgesamt bewegten Atemgasmenge an, die im Atemtrakt des Patienten aktuell verblieben ist.

Der Unterschiedswert gibt quantitativ den Atemgasmengen-Unterschied zwischen der in der früheren Atemhubmenge insgesamt zugeführten inspiratorischen Atemgasmenge und der in derselben früheren Atemhubmenge vom Patienten insgesamt abgeströmten exspiratorischen Atemgasmenge an, was unter Vernachlässigung etwaiger Leckageverluste in den Ventilen und in den Atemgas führenden Leitungen der Beatmungsvorrichtung sowie gegebenenfalls auch im Bereich des Atemtrakts selbst dem am Ende der früheren Atemhubmenge im Atemtrakt des Patienten vorhandenen Atemgas-Atemtraktinhalt entspricht.

Zur Klarstellung umfasst ein Atemhub gemäß der vorliegenden Anmeldung wenigstens einen Inspirationsvorgang, in der Regel auch einen Exspirationsvorgang. Der Exspirationsvorgang kann, wie oben beschrieben, aufgrund von Atemstapelung bzw. Doppelauslösung unvollständig sein, wobei unter die Unvollständigkeit eines Exspirationsvorgangs auch der vollständige Ausfall eines Exspirationsvorgangs fällt.

Grundsätzlich kann daran gedacht sein, den Rücksetz-Anfangswert in einem vorbestimmten Wertebereich zu wählen, welcher näher bei null als bei dem Unterschiedswert liegt. Entsprechendes gilt mutatis mutandis für den Kontinuitäts-Anfangswert, welcher in einem vorbestimmten Wertebereich gewählt werden kann, der näher bei dem Unterschiedswert als bei null liegt. Um eine Drift vollständig und sicher zu vermeiden, ist der Rücksetz-Anfangswert bevorzugt null. Zusätzlich oder alternativ ist der Kontinuitäts-Anfangswert bevorzugt der Unterschiedswert, um sicherzustellen, dass die Bestimmung des Atemtraktinhalt-Anfangswerts für den Folge-Atemhub mit der Atemtraktinhalt-Atemgasmenge am Ende der dem Folge-Atemhub unmittelbar vorhergehenden früheren Atemhubmenge als Anfangswert beginnt. Eine über eine Atemhubsgrenze fortgesetzte Integration von Atemgasflüssen, sodass die Bestimmung des Atemtraktinhalt-Anfangswerts für den Folge-Atemhub mit der Atemtraktinhalt-Atemgasmenge am Ende der dem Folge-Atemhub unmittelbar vorhergehenden früheren Atemhubmenge als Anfangswert beginnt, ist ein Setzen des Atemtraktinhalt-Anfangswerts auf den Unterschiedswert im Sinne der vorliegenden Anmeldung.

Bevorzugt wird der Atemtraktinhalt-Anfangswert während einer einheitlichen, zusammenhängenden Beatmungsbehandlung an einem Patienten wiederholt bestimmt, um einen Driftfehler während einer möglichst langen Zeitdauer der künstlichen Beatmung auszublenden, ohne jedoch dabei auch Atemstapelungen auszublenden.

Grundsätzlich kann weiter daran gedacht sein, eine etwaige Drift nur jeweils nach einer vorbestimmten Anzahl an Atemhüben zu korrigieren, sodass die frühere Atemhubmenge eine Mehrzahl von unmittelbar aufeinanderfolgenden früheren Atemhüben umfassen kann. Auf die frühere Atemhubmenge folgt ein Folge-Atemhub, für welchen der Atemtraktinhalt-Anfangswert bestimmt werden soll. Enthält die frühere Atemhubmenge jedoch zu viele Atemhübe kann ein über diese vielen Atemhübe aufsummierter Driftfehler unter Umständen von einer Atemstapelung bei ansonsten driftfreier Erfassung des Atemgas-Atemtraktinhalts nur schwierig und damit mit unerwünscht hoher Fehlerrate zu unterscheiden sein.

Um sicherzustellen, dass ein Driftfehler für die Bestimmung des Atemtraktinhalt-Anfangswerts des Folge-Atemhubs ausreichend deutlich von der Wirkung einer Atemstapelung unterscheidbar ist, gilt gemäß einer bevorzugten Weiterbildung der vorliegenden Erfindung für eine Mehrzahl von aufeinanderfolgenden Atemhüben, dass die frühere Atemhubmenge genau einen früheren Atemhub enthält. Dies ermöglicht die weitere vorteilhafte Weiterbildung, dass für jeden früheren Atemhub aus einer Mehrzahl von früheren Atemhüben abhängig vom Atemgasmengen-Unterschied ein Atemtraktinhalt-Anfangswert für den jeweiligen Folge-Atemhub bestimmt wird. In diesem Fall sind Atemhübe, die in einer Bestimmung eines Atemtraktinhalt-Anfangswerts Folge-Atemhübe sind, in einer nachfolgenden Bestimmung des Atemtraktinhalt-Anfangswerts jeweils ein früherer Atemhub.

In einer einfachen, aber robusten Ausgestaltung des Verfahrens kann der Unterschiedswert selbst als Kriterium herangezogen werden, um darüber zu entscheiden, ob der Rücksetz-Anfangswert oder der Kontinuitäts-Anfangswert als der Atemtraktinhalt-Anfangswert gesetzt wird. Eine größere Freiheit in der Gestaltung der Entscheidung für einen Atemtraktinhalt-Anfangswert und damit im Ergebnis eine höhere Genauigkeit bei der Bestimmung des Atemgas-Atemtraktinhalts kann dadurch erhalten werden, dass das Verfahren zusätzlich eine Ermittlung eines Entscheidungswerts umfasst, wobei der Entscheidungswert den Atemgasmengen-Unterschied repräsentiert.

Dabei kann es ausreichen, wenn der Entscheidungswert den Atemgasmengen-Unterschied qualitativ oder mittelbar repräsentiert. Beispielsweise kann der Entscheidungswert den Atemgasmengen-Unterschied anhand eines Unterschieds der zeitlichen Dauer des wenigstens einen Inspirationsvorgangs und der zeitlichen Dauer des wenigstens einen Exspirationsvorgangs der früheren Atemhubmenge beurteilen und aus dem Unterschied der genannten Zeitdauern auf einen Unterschied der zugehörigen inspiratorischen und exspiratorischen Atemgasmengen schließen. Der Entscheidungswert kann eine vorbestimmte Funktion der zeitlichen Dauer des wenigstens einen Inspirationsvorgangs und der zeitlichen Dauer des wenigstens einen Exspirationsvorgangs der früheren Atemhubmenge sein. Der Entscheidungswert kann beispielsweise proportional zu einem Quotienten aus der zeitlichen Dauer des wenigstens einen Inspirationsvorgangs und der zeitlichen Dauer des wenigstens einen Exspirationsvorgangs der früheren Atemhubmenge sein.

Eine besonders genaue Entscheidung für den einen oder den anderen Atemtraktinhalt-Anfangswert kann dann getroffen werden, wenn der Entscheidungswert den Unterschiedswert repräsentiert. Der Entscheidungswert kann eine vorbestimmte Funktion des Unterschiedswerts sein. Vorteilhafte Ausgestaltungen des Entscheidungswerts werden weiter unten diskutiert. Bei der Bestimmung des Atemtraktinhalt-Anfangswerts kann dann abhängig vom Entscheidungswert der Rücksetz-Anfangswert oder der Kontinuitäts-Anfangswert als der Atemtraktinhalt-Anfangswert bestimmt werden.

Dann, wenn wie oben beschrieben für eine Mehrzahl von aufeinanderfolgenden Atemhüben gilt, dass die frühere Atemhubmenge genau einen früheren Atemhub enthält, kann folglich für jeden aus einer Mehrzahl von früheren Atemhüben ein Entscheidungswert ermittelt und abhängig vom jeweiligen Entscheidungswert für jeden auf einen früheren Atemhub aus der Mehrzahl von früheren Atemhüben folgenden Folge-Atemhub ein Atemtraktinhalt-Anfangswert bestimmt werden.

Die Verwendung eines vom Unterschiedswert betragsmäßig verschiedenen, aber den Unterschiedswert repräsentierenden Entscheidungswerts ermöglicht es beispielsweise, in für die Aussagekraft des Entscheidungswerts vorteilhafter Weise den Unterschiedswert in Beziehung zu der während der früheren Atemhubmenge insgesamt bewegten Atemgasmenge zu setzen. Denn ein gegebener absoluter Parameter einer Beatmungsbehandlung, wie etwa Teilatemhubdauer in Sekunden oder Unterschiedswert in Volumen, Masse oder Gewicht ist medizinisch abhängig davon unterschiedlich zu bewerten, ob er bei einem beatmeten Kleinkind oder bei einem beatmeten Erwachsenen aufgetreten ist.

Bevorzugt setzt der Entscheidungswert den Unterschiedswert in Beziehung zu der dem Patienten während der früheren Atemhubmenge zugeführten inspiratorischen Atemgasmenge. Der Entscheidungswert kann daher proportional zu einem Quotienten aus dem Unterschiedswert und einer dem Patienten unter Beteiligung der Beatmungsvorrichtung während der früheren Atemhubmenge zugeführten inspiratorischen Atemgasmenge sein. Der Proportionalitätsfaktor kann nach Maßgabe der Charakteristik der jeweils verwendeten Beatmungsvorrichtung gewählt sein, etwa unter Berücksichtigung eines bei jedem Atemhub erfolgenden Leckageverlusts. Es reicht jedoch aus, für den Proportionalitätsfaktor den Wert 1 zu wählen, sodass der Entscheidungswert gleich dem oben genannten Quotienten ist. Als Quotient gilt auch sein Kehrwert, welcher den gleichen Informationsgehalt und somit die gleiche Aussagekraft besitzt. Wie oben bereits angedeutet, kann der Entscheidungswert der Unterschiedswert selbst sein, wenngleich dies aus den vorgenannten Gründen nicht bevorzugt ist.

Die Bestimmung des Atemtraktinhalt-Anfangswerts abhängig vom Entscheidungswert kann in vorteilhafter Weise einen Vergleich des Entscheidungswerts mit einem vorbestimmten Entscheidungs-Schwellenwert umfassen, wobei der Atemtraktinhalt-Anfangswert abhängig von einem Ergebnis des Vergleichs bestimmt wird. Der Entscheidungs-Schwellenwert kann nach Maßgabe einer Versuchsreihe an der verwendeten Beatmungsvorrichtung so gewählt werden, dass die Fehlerrate an unerwünscht falschpositiv erkannten, tatsächlich jedoch nicht aufgetretenen Atemstapelungen oder/und an unerwünscht falsch-negativ unerkannt gebliebenen tatsächlichen Atemstapelungen möglichst gering ist.

Bei der bevorzugten wiederholten Bestimmung eines Atemtraktinhalt-Anfangswerts während einer künstlichen Beatmung können Artefakte auftreten, welche mit fortschreitender Zeit ein zunehmend unrealistisches Bild von der tatsächlich während der künstlichen Beatmung bewegten Atemgasmenge zeichnen.

Beispielsweise weiß die Anmelderin aus medizinischen Beobachtungen, dass Atemstapelungen nicht beliebig oft hintereinander auftreten können, da es das körperlich begrenzte Atemtraktvolumen eines Patienten unmöglich macht, einem unvollständig ausatmenden Patienten beliebig häufig hintereinander das an der Beatmungsvorrichtung eingestellte Tidalvolumen zu verabreichen. Daher kann in dem vorliegend vorgeschlagenen Verfahren vorgesehen sein, bei mehrfacher Bestimmung eines Atemtraktinhalt-Anfangswerts für unterschiedliche Atemhübe dann den Rücksetz-Anfangswert unabhängig vom Atemgasmengen-Unterschied, insbesondere unabhängig vom Entscheidungswert, als den Atemtraktinhalt-Anfangswert zu setzen, wenn zuvor bei einer vorbestimmten ersten Anzahl von aufeinander folgenden Bestimmungen des Atemtraktinhalt-Anfangswerts jedes Mal der Kontinuitäts-Anfangswert als der Atemtraktinhalt-Anfangswert gesetzt wurde. In bisherigen Untersuchungen hat sich 4 als eine geeignete vorbestimmte erste Anzahl erwiesen, wenngleich die erste Anzahl auch 3 oder 5 oder sogar 6 sein kann.

Unter dem gleichen Aspekt einer physiologisch unmöglichen zu häufigen Verabreichung des Tidalvolumens ohne zwischenzeitliche ausreichende Exspiration kann der Rücksetz-Anfangswert dann unabhängig vom Atemgasmengen-Unterschied, insbesondere unabhängig vom Entscheidungswert, als der Atemtraktinhalt-Anfangswert gesetzt werden, wenn zuvor innerhalb einer vorbestimmten zweiten Anzahl von Bestimmungen des Atemtraktinhalt-Anfangswerts die Anzahl von Bestimmungen, die den Kontinuitäts-Anfangswert zum Ergebnis hatten, einen vorbestimmten Anteils-Schwellenwert erreicht oder übersteigt, wobei der Anteils-Schwellenwert kleiner als die zweite Anzahl ist. Ebenso ist die zweite Anzahl bevorzugt größer als die erste Anzahl. Der Anteils-Schwellenwert kann ein prozentualer Schwellenwert oder ein absoluter Schwellenwert sein. Beispielsweise kann der Anteils-Schwellenwert 50 % sein oder kann der Anteils-Schwellenwert 5 Bestimmungen bei einer zweiten Anzahl von 10 Bestimmungen betragen. Bei einem prozentual definierten Anteils-Schwellenwert lässt sich die zweite Anzahl an Bestimmungen als Bezugsgröße in einem die Ausführung des vorgeschlagenen Verfahrens bewirkenden Datenverarbeitungsprogramm mit geringerem Aufwand verändern als bei einem absolut definierten Anteils-Schwellenwert.

Ebenso kann alternativ oder bevorzugt zusätzlich vorgesehen sein, dass der Rücksetz-Anfangswert dann unabhängig vom Atemgasmengen-Unterschied, insbesondere unabhängig vom Entscheidungswert, als der Atemtraktinhalt-Anfangswert bestimmt wird, wenn innerhalb einer vorbestimmten Anzahl von zuvor ausgeführten Atemhüben gilt, dass eine Häufigkeit, mit welcher ein Quotient aus einem ersten Atemtraktinhalt-Unterschied und einem vom ersten verschiedenen zweiten Atemtraktinhalt-Unterschied einen vorbestimmten Quotienten-Schwellenwert erreicht oder übersteigt, einen Häufigkeits-Schwellenwert erreicht oder übersteigt. Der erste Atemtraktinhalt-Unterschied ist bevorzugt ein Unterschied zwischen dem Atemgas-Atemtraktinhalt am Ende eines bestimmten Atemhubs und dem Atemtraktinhalt an Atemgas am Ende einer dem bestimmten Atemhub vorausgehenden früheren Atemhubmenge. Der zweite Atemtraktinhalt-Unterschied ist bevorzugt ein Unterschied zwischen einem Atemtraktinhalt an Atemgas am Ende einer Inspirationsphase des bestimmten Atemhubs und dem Atemtraktinhalt an Atemgas am Ende der dem bestimmten Atemhub vorausgehenden früheren Atemhubmenge. Formelhaft ausgedrückt bedeutet dies für ein bevorzugtes Ausführungsbeispiel:

$$\frac{V_{endexp,i} - V_{endexp,i-1}}{V_{endinsp,i} - V_{endexp,i-1}} \geq QS \qquad \text{Gl. 1}$$

Der Inkrement- oder Zählindex i bezieht sich auf den späteren betrachteten Atemhub, also in der obigen Terminologie den bestimmten Atemhub innerhalb der vorbestimmten Anzahl an zuvor ausgeführten Atemhüben. Der bestimmte Atemhub ist frei wählbar, sollte jedoch nicht der erste Atemhub des Beatmungsvorgangs sein, da ihm ein Atemhub vorausgegangen sein muss. Bevorzugt ist der bestimmte Atemhub der unmittelbar dem Folge-Atemhub vorausgehende Atemhub.

Der Inkrement- oder Zählindex i-1 bezieht sich somit auf die dem bestimmten Atemhub unmittelbar vorhergehende frühere Atemhubmenge, insbesondere auf den unmittelbar vorhergehenden früheren Atemhub. In der Gleichung 1 bezeichnet $V_{endexp,i}$ den Atemgas-Atemtraktinhalt am Ende eines bestimmten Atemhubs, also am Ende der Exspirationsphase des bestimmten Atemhubs; $V_{endexp,i-1}$ bezeichnet den Atemgas-Atemtraktinhalt am Ende der dem bestimmten Atemhub vorausgehenden früheren Atemhubmenge, insbesondere eines früheren Atemhubs, also wiederum am Ende einer Exspirationsphase; $V_{endinsp,i}$ bezeichnet den Atemgas-Atemtraktinhalt am Ende einer Inspirationsphase des Folge-Atemhubs und QS ist der Quotienten-Schwellenwert. Der Quotienten-Schwellenwert kann auf Grundlage experimenteller Untersuchungen ermittelt werden. Er liegt bevorzugt zwischen 0,1 und 0,3, bevorzugt zwischen 0,15 und 0,25 und besonders bevorzugt bei 0,2.

Der Häufigkeits-Schwellenwert kann als prozentualer Schwellenwert etwa 40 % bis 80 % betragen. Bevorzugt liegt der Häufigkeits-Schwellenwert in einem Bereich zwischen 40 % und 60 %, besonders bevorzugt bei 50 %.

Die vorbestimmte Häufigkeit kann zwischen 5 und 20 Atemhübe betragen, bevorzugt zwischen 8 und 15 Atemhübe, besonders bevorzugt 10 Atemhübe.

Eine weitere Möglichkeit einer Fehlbeurteilung des Atemgas-Atemtraktinhalts kann auf einer während der künstlichen Beatmung erfolgten Änderung des positiven endexspiratorischen Drucks (PEEP = Positive End-Expiratory Pressure) beruhen. Eine Änderung des PEEP bedeutet aufgrund der bekannten Zusammenhänge zwischen Druck und Volumen an Gasen auch eine Änderung des endexspiratorischen Atemgas-Atemtraktinhalts. Zur vorteilhaften Vermeidung einer durch eine Änderung des PEEP verursachten Fehlbeurteilung des Atemgas-Atemtraktinhalts kann im Rahmen des vorgeschlagenen Verfahrens vorgesehen sein, dass der Rücksetz-Anfangswert dann unabhängig vom Atemgasmengen-Unterschied, insbesondere unabhängig vom Entscheidungswert, als der Atemtraktinhalt-Anfangswert bestimmt wird, wenn innerhalb einer vorbestimmten Zeitspanne vor der aktuellen Bestimmung des Atemtraktinhalt-Anfangswerts eine quantitative Änderung des PEEP erfasst wurde. Die vorbestimmte Zeitspanne kann relativ, bezogen auf eine Bezugszeitspanne, definiert sein. Die vorbestimmte Zeitspanne oder/und die Bezugszeitspanne kann bzw. können absolut als Zeitspanne in Sekunden, Minuten und dergleichen definiert sein. Die vorbestimmte Zeitspanne oder/und die Bezugszeitspanne kann bzw. können jedoch ebenso funktional, etwa durch die Dauer einer vorbestimmten Anzahl früherer Atemhübe oder Atemhubabschnitte definiert sein. Ein Atemhubabschnitt kann beispielsweise die Inspirationsphase oder die Exspirationsphase des Atemhubs sein.

Weiterhin treten während einer künstlichen Beatmung, insbesondere nach einer Atemstapelung, aber auch sonst, gelegentlich überdurchschnittlich tiefe Exspirationsvorgänge auf, bei welchen der Patient mehr exspiratorisches Atemgas abgibt als er während der vorhergehenden Inspirationsphase aufgenommen hat. Der Atemtrakt des Patienten ist nach einer solchen tiefen Exspiration mit einem überdurchschnittlichen exspiratorischen Atemgasvolumen mit größter Wahrscheinlichkeit nicht in medizinisch-kritischem Umfang mit Atemgas teilgefüllt. Daher kann der Rücksetz-Anfangswert dann unabhängig vom Atemgasmengen-Unterschied, insbesondere unabhängig vom Entscheidungswert, als der Atemtraktinhalt-Anfangswert bestimmt werden, wenn für wenigstens eine der beiden dem aktuellen Folge-Atemhub, für welchen der Atemtraktinhalt-Anfangswert bestimmt wird, unmittelbar vorausgehenden früheren Atemhubmengen, insbesondere für wenigstens einen der beiden unmittelbar vorausgehenden früheren Atemhübe, ermittelt wird, dass ein aus den erfassten exspiratorischen Atemgasflüssen ermitteltes exspiratorisches Atemgasvolumen der früheren Atemhubmenge betragsmäßig größer ist als ein aus den inspiratorischen Atemgasflüssen ermitteltes inspiratorisches Atemgasvolumen derselben früheren Atemhubmenge. Dies bedeutet in einer bevorzugten Ausführungsform zum einen, dass unmittelbar nach einer oben genannten tiefen Exspiration die Bestimmung des Atemtraktinhalts mit dem Rücksetz-Anfangswert beginnen kann, da in diesem Fall keine Atemstapelung droht. Dies bedeutet in einer bevorzugten Ausführungsform zum anderen, dass die Bestimmung des Atemtraktinhalts unmittelbar nach einer unvollständigen Exspiration selbst dann mit dem Rücksetz-Anfangswert beginnen kann, wenn unmittelbar vor der unvollständigen Exspiration eine tiefe Exspiration stattgefunden hat. In diesem zweitgenannten Fall findet zwar eine Atemstapelung statt, aber an einem zuvor über-exspirierten Atemtrakt, sodass selbst nach der unvollständigen Exspiration durch die Atemstapelung keine übermäßige Belastung des Atemtrakts zu befürchten ist.

Zur erleichterten Überwachung des Beatmungsvorgangs kann der Atemtraktinhalt an Atemgas graphisch als Funktion der Zeit an einer Ausgabevorrichtung ausgegeben werden.

Die oben genannte objektive technische Aufgabe wird auch gelöst durch eine Beatmungsvorrichtung zur wenigstens teilweise künstlichen Beatmung eines Patienten, umfassend:

- eine Atemgas-Quellenanordnung, welche ein inspiratorisches Atemgas zur künstlichen Beatmung des Patienten bereitstellt,
- eine Strömungsveränderungsvorrichtung, welche dazu ausgebildet ist, einen inspiratorischen Atemgasfluss zu erzeugen und betragsmäßig zu verändern,
- eine Atemgasleitungsanordnung mit einem im Betrieb dem Patienten näher gelegenen proximalen Längsende und mit einem im Betrieb vom Patienten weiter entfernt gelegenen distalen Längsende, um den inspiratorischen Atemgasfluss von der Atemgas-Quellenanordnung zum Patienten hin zu fördern,
- eine Flusssensoranordnung, welche dazu ausgebildet ist, den inspiratorischen Atemgasfluss ebenso wie einen exspiratorischen Atemgasfluss betragsmäßig zu erfassen,
- eine Steuervorrichtung mit einem Datenspeicher, wobei die Steuervorrichtung signalübertragungsmäßig mit dem Datenspeicher und mit der Flusssensoranordnung verbunden ist und welche dazu ausgebildet ist, die Betriebsleistung der Strömungsveränderungsvorrichtung zur Veränderung des inspiratorischen Atemgasflusses zu steuern,

wobei die Steuervorrichtung dazu ausgebildet ist, das oben beschriebene und vorteilhaft weitergebildete Verfahren auszuführen. Die Steuervorrichtung kann einen Prozessor mit integrierten Schaltkreisen und einen Datenspeicher mit einem darauf gespeicherten, vom Prozessor abrufbaren und abarbeitbaren Programm aufweisen, wobei die Ausführung des Programms durch den Prozessor zur Durchführung des oben beschriebenen Verfahrens führt.

Die Atemgas-Quellenanordnung der Beatmungsvorrichtung kann als eine Atemgasquelle eine Ansaugöffnung aufweisen, durch welche hindurch Umgebungsluft oder Gas aus einem vorbestimmten Gasvorrat angesaugt werden kann. Die Atemgas-Quellenanordnung kann zusätzlich oder alternativ einen Gasvorrat als Atemgasquelle aufweisen, beispielsweise als Vorratsbehälter oder als Anschlussformation zum Anschluss einer Versorgungsleitung, welche die Beatmungsvorrichtung mit einem lokal installierten Gasvorrat verbindet, wie dies häufig in Kliniken der Fall ist. Um die Möglichkeit bereitzustellen, unterschiedliche Gase zu einem Atemgas zu mischen, kann die Atemgas-Quellenanordnung eine Mehrzahl von einzelnen Atemgasquellen, wie die oben genannten, aufweisen. Dabei können die unterschiedlichen zu mischenden Gase aufgrund ihrer individuellen Bereitstellung und Entspannung unterschiedliche Temperaturen oder/und unterschiedliche Feuchtigkeit aufweisen. Um sicherzugehen, dass das inspiratorische Atemgas den Patienten mit einer einmal eingestellten Feuchtigkeit tatsächlich erreicht, wird besonders bevorzugt in Inspirationsrichtung stromabwärts einer bevorzugt vorhandenen Befeuchtungsvorrichtung kein Atemgasbestandteil mehr dem aus der Befeuchtungsvorrichtung austretenden Atemgasfluss zugefügt.

Die Flusssensoranordnung kann einen oder mehrere Flusssensoren aufweisen, etwa je einen für den inspiratorischen und für den exspiratorischen Atemgasfluss. Bevorzugt umfasst die Flusssensoranordnung nur einen Flusssensor, um sowohl den inspiratorischen als auch den exspiratorischen Atemgasfluss zu erfassen. Dieser ist bevorzugt proximal zwischen Atemgasleitungsanordnung und einer Patientenschnittstelle angeordnet, kann aber auch distal in einem Gehäuse der Beatmungsvorrichtung aufgenommen sein, in welchem beispielsweise auch die Strömungsveränderungsvorrichtung aufgenommen ist. Zur Erzielung von höherer Prozesssicherheit kann die Beatmungsvorrichtung auch mehrere Flusssensoren aufweisen, die jeweils sowohl den inspiratorischen als auch den exspiratorischen Atemgasfluss erfassen, etwa einen distalen Flusssensor in einem Gehäuse der Beatmungsvorrichtung und einen proximalen Flusssensor nahe beim Patienten.

Bevorzugt weist die Beatmungsvorrichtung eine Drucksensoranordnung auf, die ebenfalls einen oder mehrere Drucksensoren aufweisen kann, um den Druck des inspiratorischen oder/und des exspiratorischen Atemgases zu messen.

Bevorzugt umfasst die Flusssensoranordnung einen Differenzdruck-Flusssensor, so dass mit dem Differenzdruck-Flusssensor am Ort der Erfassung des Atemgasflusses auch eine Erfassung des jeweils herrschenden Atemgasdrucks möglich ist. Besonders bevorzugt ist die Flusssensoranordnung eine proximale, patientennahe Flusssensoranordnung. Beispielsweise ist die proximale Flusssensoranordnung, mit welcher die Atemgasflüsse zur Bestimmung der Atemtraktinhalt-Anfangswerte erfasst werden, nicht weiter als 80 cm, vorzugsweise nicht weiter als 50 cm, vom Mund des Patienten entfernt. Eine proximale Flusssensoranordnung weist eine bessere Synchronität als eine distale, weiter vom Patienten entfernte Flusssensoranordnung auf und gestattet so eine genauere Erfassung eines vom Patienten verursachten Triggerns eines Inspirationsvorgangs, also einer Inspirationsanstrengung des Patienten.

Bevorzugt umfasst die Beatmungsvorrichtung, insbesondere die Steuervorrichtung, auch eine Zeitmessvorrichtung, um Zeitdauern von Vorgängen und Teilvorgängen während einer Beatmungsbehandlung ermitteln zu können.

Zur Realisierung der graphischen Ausgabe des bestimmten Atemgas-Atemtraktinhalts als Funktion der Zeit weist die Beatmungsvorrichtung bevorzugt eine graphische Ausgabevorrichtung auf, etwa einen Monitor oder einen Touchscreen. Die Steuervorrichtung ist dann bevorzugt dazu ausgebildet, den bestimmten Atemgas-Atemtraktinhalt als Funktion der Zeit graphisch an der Ausgabevorrichtung auszugeben.

Um einem die künstliche Beatmung eines Patienten begleitenden Therapeuten die einfache Möglichkeit zu geben, eine potentiell kritische Atemstapelung schnell und sicher zu erkennen, kann die graphische Ausgabe des Atemtraktinhalts an Atemgas als Funktion der Zeit an der Ausgabevorrichtung die Maßnahme umfassen, dann, wenn der Kontinuitäts-Anfangswert als der Atemtraktinhalt-Anfangswert gesetzt wird, die graphische Ausgabe des Atemtraktinhalts als Funktion der Zeit zu verändern.

Die Änderung der graphischen Ausgabe kann eine Änderung des Graphen des Atemtraktsinhalts als Funktion der Zeit sein, etwa eine Änderung der Linienfarbe oder/und der Linienstärke, etwa zwischen dünner und dicker Linienstärke, oder/und der Linienart, etwa zwischen punktierter oder/und strichlinierter oder/und durchgehender Linie.

Zusätzlich oder alternativ kann die Änderung der graphischen Ausgabe eine Änderung wenigstens eines Abschnitts des Hintergrunds sein, auf welchem der Graph des Atemtraktsinhalts als Funktion der Zeit ausgegeben wird. Beispielsweise kann die Farbe oder/und die Textur, etwa zwischen schraffiert und uni, des Hintergrunds geändert werden. Dabei kann entweder der gesamte Hintergrund geändert werden oder nur ein zwischen dem Graphen und einer Bezugslinie, etwa der Null-Linie des Atemtraktinhalts, geändert werden.

Grundsätzlich kann die Änderung der graphischen Ausgabe eine Änderung der gesamten graphischen Ausgabe sein, auch von Teilen die den zeitlichen Verlauf des Atemtraktinhalts der Vergangenheit darstellen. Dies gilt insbesondere, aber nicht nur, für eine Änderung des Hintergrunds.

Ein erleichtertes Erkennen nicht nur dass eine Atemstapelung stattgefunden hat, sondern auch wann dies geschehen ist, kann dem begleitenden Therapeuten in vorteilhafter Weise dadurch ermöglicht werden, dass die Änderung in der graphischen Ausgabe erst ab dem Zeitpunkt der Auswahl des Kontinuitäts-Anfangswerts als der Atemtraktinhalt-Anfangswert ausgeführt wird und bereits ausgegebene graphische Inhalte, die immer noch dargestellt werden, unverändert bleiben. Die Beatmungsvorrichtung ist entsprechend zur Durchführung dieser veränderten graphischen Ausgabe ausgebildet.

Zur Eingabe und ganz allgemein zur Ausgabe von Daten umfasst die Beatmungsvorrichtung bevorzugt eine von der Steuervorrichtung steuerbare Eingabe/Ausgabevorrichtung, zu der auch die oben genannte graphische Ausgabevorrichtung gehören kann. Die Eingabe/Ausgabevorrichtung kann eine Mehrzahl von Tasten, Drehschaltern, einen Touchscreen, einen Lautsprecher, Leuchtmittel und dergleichen umfassen. Ebenso kann die Beatmungsvorrichtung Datenschnittstellen oder/und Datenübertragungsleitungen aufweisen, um Daten von anderen Vorrichtungen zu erhalten oder/und an diese zu übertragen. Datenschnittstellen können Buchsen, Funkantennen, Stecker und dergleichen sein.

Grundsätzlich ist die Beatmungsvorrichtung bevorzugt dazu ausgebildet, einen Patienten gemäß unterschiedlicher Beatmungsmodi zu beatmen, aus welchen der behandelnde Arzt bei der Einstellung der Beatmungsvorrichtung auswählen kann. Beispielsweise kann die Beatmungsvorrichtung einen Patienten volumenbezogen gesteuert oder druckbezogen gesteuert künstlich beatmen. Da Atemstapelungen bzw. Doppelauslösungen vor allem bei Durchführung von unterstützenden bzw. Unterstützungs-Beatmungsmodi auftreten, bei welchen ein Patient durch eine Inspirationsanstrengung eine dann von der Beatmungsvorrichtung unterstützte künstliche Inspiration auslösen kann, ist die Beatmungsvorrichtung bevorzugt dazu ausgebildet, eine künstliche Beatmung in einem Unterstützungs-Beatmungsmodus durchzuführen, in welchem die Steuervorrichtung eine Inspirationsanstrengung des beatmeten Patienten erfasst und auf die Erfassung der Inspirationsanstrengung hin die Strömungsveränderungsvorrichtung ansteuert, dem Patienten eine inspiratorische Atemgasmenge über die Atemgasleitungsanordnung zu verabreichen. Eine Inspirationsanstrengung kann beispielsweise durch Überwachung eines Atemgasflusses oder/und eines Atemgasdruckes in der Atemgas-Leitungsanordnung auf plötzliche typische Änderungen erfasst werden.

Die Steuervorrichtung ist zur Milderung der Auswirkungen unerwünschter Atemstapelungen bzw. Doppelauslösungen bevorzugt dazu ausgebildet, auf Grundlage des verfahrensgemäß bestimmten Atemtraktinhalt-Anfangswerts oder/und auf Grundlage des bestimmten Atemtraktinhalts an Atemgas, eine unvollständige Exspiration zu erkennen. Dann, wenn der Kontinuitäts-Anfangswert als der Atemtraktinhalt-Anfangswert gesetzt wird, kann eine unvollständige Exspiration durch Überschreiten von einem oder mehreren Schwellenwerten für den Atemgas-Atemtraktinhalt während einer nachfolgenden Inspiration einfach und sicher erkannt werden. Bevorzugt ist die Steuervorrichtung dazu ausgebildet, dann, wenn die Steuervorrichtung eine unvollständige Exspiration erkannt hat, den Beatmungsmodus zu ändern, insbesondere zwischen einem volumenbezogen gesteuerten Beatmungsmodus und einem druckbezogen gesteuerten Beatmungsmodus zu wechseln. Von besonderem Interesse ist dabei der Wechsel von einem volumenbezogen gesteuerten Beatmungsmodus, welche den unvollständig exspirierten Atemtrakt zu "überfüllen" droht, zu einem druckbezogen gesteuerten Beatmungsmodus, welcher beispielsweise auf einen während einer Inspiration zu erreichenden Atemgas-Druckwert steuert und nicht auf die Zufuhr eines vorbestimmten Tidalvolumens.

Die zuvor bezeichnete Atemgasmenge kann eine Masse, ein Gewicht oder ein Volumen an Atemgas sein. Bevorzugt ist die Atemgasmenge, wie in der medizinischen Beatmungstechnik bevorzugt und üblich, ein Atemgasvolumen.

Die vorliegende Erfindung wird nachfolgend anhand der beiliegenden Zeichnungen näher erläutert werden. Es stellt dar:

Figur 1    eine schematische Darstellung einer erfindungsgemäßen, zur künstlichen Beatmung eines Patienten hergerichteten Beatmungsvorrichtung, und

Figur 2    eine graphische Ausgabe eines zeitlichen Verlaufs des Atemgas-Atemtraktinhalts eines künstlich beatmeten Patienten, je einmal erstellt mit einem herkömmlichen Bestimmungsverfahren und einmal mit dem vorliegend vorgeschlagenen Verfahren, und

Figur 3    ein Flussdiagramm eines Ablaufs einer erfindungsgemäßen Ausführungsform des vorliegend vorgeschlagenen Verfahrens.

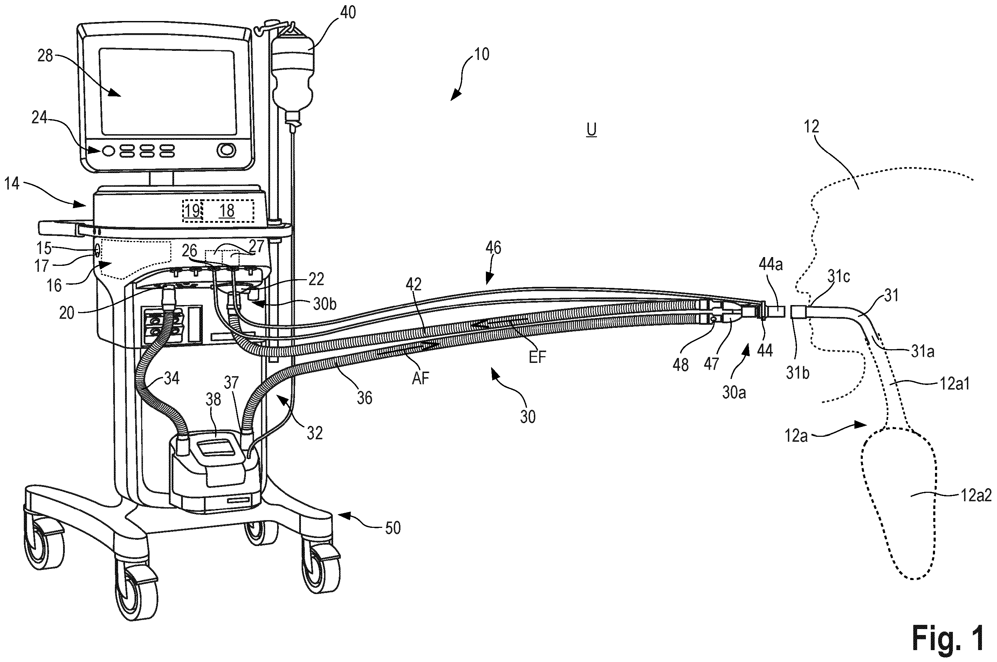

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines, bevorzugt humanen, Patienten 12.

Die Beatmungsvorrichtung 10 weist ein Gehäuse 14 auf, in welchem eine Ansaugöffnung 15 ausgebildet und - von außen wegen des blickdichten Gehäusematerials nicht erkennbar - eine Strömungsveränderungsvorrichtung 16 sowie eine Steuervorrichtung 18 aufgenommen sind. Die Ansaugöffnung 15 gestattet der Strömungsveränderungsvorrichtung 16, Umgebungsluft aus der Außenumgebung U der Beatmungsvorrichtung anzusaugen und nach an sich bekannter Reinigung durch Filter als Atemgas dem Patienten 12 zuzuführen. Die Ansaugöffnung 15 ist daher eine Atemgas-Quellenanordnung im Sinne der vorliegenden Anmeldung.

In der Ansaugöffnung 15 kann sich ein Umgebungstemperatursensor 17 befinden, welcher die Temperatur der Luft der Umgebung U misst und an die Steuervorrichtung 18 überträgt.

Die Strömungsveränderungsvorrichtung 16 ist in an sich bekannter Weise aufgebaut und kann eine Pumpe oder/und einen Verdichter oder/und ein Gebläse oder/und einen Druckbehälter oder/und ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuervorrichtung 18 ist üblicherweise als Computer oder Mikroprozessor realisiert. Sie umfasst einen in Figur 1 mit 19 bezeichneten Datenspeicher, um für den Betrieb der Beatmungsvorrichtung 10 notwendige Daten speichern und erforderlichenfalls abrufen zu können. Der Datenspeicher 19 kann bei Netzwerkbetrieb auch außerhalb des Gehäuses 14 gelegen und durch eine Datenübertragungsverbindung mit der Steuervorrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist der Datenspeicher 19 bevorzugt in die Steuervorrichtung 18 integriert oder wenigstens im selben Gehäuse 14 wie diese aufgenommen.

Zur Eingabe von Daten in die Beatmungsvorrichtung 10 bzw. genauer in die Steuervorrichtung 18 kann die Beatmungsvorrichtung 10 eine Eingabevorrichtung 24 aufweisen, welche in dem in Figur 1 dargestellten Beispiel durch eine Tastatur repräsentiert ist. Wie weiter unten noch erläutert werden wird, ist die Tastatur nicht notwendigerweise der einzige Dateneingang der Steuervorrichtung 18. Tatsächlich kann die Steuervorrichtung 18 zusätzlich oder alternativ zur Tastatur Daten über verschiedene Dateneingänge erhalten, etwa über eine Netzwerkleitung, eine Funkstrecke oder über Sensoranschlüsse 26.

Zur Ausgabe von Daten an den behandelnden Therapeuten kann die Beatmungsvorrichtung 10 ein Ausgabegerät 28 aufweisen, im dargestellten Beispiel einen Monitor.

Zur künstlichen Beatmung ist der Patient 12 mit der Beatmungsvorrichtung 10, genauer mit der Strömungsveränderungsvorrichtung 16 im Gehäuse 14, über eine Atemgasleitungsanordnung 30 verbunden. Der Patient 12 ist hierfür mittels eines Endotrachealtubus als einer Patientenschnittstelle 31 intubiert. Ein proximales Längsende 31a der Patientenschnittstelle 31 gibt den inspiratorischen Atemgasfluss AF in den Atemtrakt 12a des Patienten 12 ab. Durch das proximale Längsende 31a strömt auch der exspiratorische Atemgasstrom EF in die Atemgasleitungsanordnung 30 ein.

Der Atemtrakt 12a des Patienten 12 umfasst dessen obere Atemwege 12a1 und dessen untere Atemwege 12a2. Das gemeinhin als "Lunge" bezeichnete Körperorgan zählt zu den unteren Atemwegen 12a2.

Ein distales Längsende 31b der Patientenschnittstelle 31 ist zur Verbindung mit der Atemgasleitungsanordnung 30 ausgebildet. Ab dem Ort 31c in Inspirationsrichtung stromabwärts bis zum proximalen Längsende 31a ist die Patientenschnittstelle vom Körper des Patienten 12 umgeben. Dies bedeutet im Umkehrschluss, dass die Patientenschnittstelle 31 von seinem distalen Längsende 31b bis zum Ort 31c der Außenumgebung U ausgesetzt ist und mit dieser in, überwiegend konvektiver, Wärmeübertragungsverbindung steht.

Die Atemgasleitungsanordnung 30 weist einen Inspirationsschlauch 32 auf, über welchen frisches Atemgas von der Strömungsveränderungsvorrichtung 16 in den Atemtrakt 12a des Patienten 12 geleitet werden kann. Der Inspirationsschlauch 32 kann unterbrochen sein und einen ersten Inspirationsschlauch 34 und einen zweiten Inspirationsschlauch 36 aufweisen, zwischen welchen eine Befeuchtungsvorrichtung 38 zur gezielten Befeuchtung und gegebenenfalls auch Temperierung des dem Patienten 12 zugeführten inspiratorischen Atemgases vorgesehen sein kann. Die Befeuchtungsvorrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, der Befeuchtungsvorrichtung 38 zugeführt werden kann. Bei einem Einsatz der vorliegenden Beatmungsvorrichtung 10 als Anästhesie-Beatmungsvorrichtung können auf diese Weise volatile Anästhetika kontrolliert über die Beatmungsvorrichtung 10 an den Patienten 12 abgegeben werden. Die Befeuchtungsvorrichtung 38 sorgt dafür, dass das frische Atemgas dem Patienten 12 mit einer vorbestimmten Feuchtigkeit, gegebenenfalls unter Zugabe eines Medikamenten-Aerosols, und mit einer vorbestimmten Temperatur zugeführt wird.

Der zweite Inspirationsschlauch 36 ist im vorliegenden Beispiel durch eine Leitungsheizvorrichtung 37 elektrisch beheizbar. Die Leitungsheizvorrichtung 37 ist durch die Steuervorrichtung 18 zum Betrieb ansteuerbar. Abweichend vom Gesagten kann auch der erste Inspirationsschlauch 34 beheizbar sein oder/und kann der wenigstens eine Schlauch 34 oder/und 36 durch eine andere als eine elektrische Leitungsheizvorrichtung 37 beheizbar sein, etwa durch Umspülung mit einem Wärmetauschmedium.

Die Atemgasleitungsanordnung 30 weist neben dem bereits erwähnten Inspirationsventil 20 und Exspirationsventil 22 weiter einen Exspirationsschlauch 42 auf, über welchen verstoffwechseltes Atemgas als exspiratorischer Atemgasfluss EF aus dem Atemtrakt 12a des Patienten 12 in die Außenumgebung U abgeblasen wird.

Am distalen Längsende 30b der Atemgasleitungsanordnung 30 sind der Inspirationsschlauch 32 mit dem Inspirationsventil 20 und der Exspirationsschlauch 42 mit dem Exspirationsventil 22 gekoppelt. Von den beiden Ventilen ist vorzugsweise jeweils nur eines gleichzeitig zum Durchlass einer Gasströmung geöffnet. Die Betätigungssteuerung der Ventile 20 und 22 erfolgt ebenfalls durch die Steuervorrichtung 18.

Während eines Beatmungszyklus ist zunächst für die Dauer der Inspirationsphase das Exspirationsventil 22 geschlossen und das Inspirationsventil 20 geöffnet, sodass frisches inspiratorisches Atemgas vom Gehäuse 14 zum Patienten 12 geleitet werden kann. Eine Strömung des frischen Atemgases wird durch gezielte Druckerhöhung des Atemgases durch die Strömungsveränderungsvorrichtung 16 bewirkt. Aufgrund der Druckerhöhung strömt das frische Atemgas in den Atemtrakt 12a des Patienten 12 und expandiert dort den atemtraktnahen Körperbereich, also insbesondere den Brustkorb, gegen die individuelle Elastizität der atemtraktnahen Körperteile. Hierdurch steigt auch der Gasdruck im Inneren des Atemtrakts 12a des Patienten 12 an.

Am Ende der Inspirationsphase werden das Inspirationsventil 20 geschlossen und das Exspirationsventil 22 geöffnet. Es beginnt die Exspirationsphase. Aufgrund des bis zum Ende der Inspirationsphase erhöhten Gasdrucks des im Atemtrakt 12a des Patienten 12 vorhandenen Atemgases strömt dieses nach dem Öffnen des Exspirationsventils 22 in die Außenumgebung U, wobei sich mit fortschreitender Strömungsdauer der Gasdruck im Atemtrakt 12a des Patienten 12 verringert. Erreicht der Gasdruck im Atemtrakt 12a einen an der Beatmungsvorrichtung 10 eingestellten positiven end-exspiratorischen Druck (PEEP), also einen geringfügig höheren Druck als den Atmosphärendruck, wird die Exspirationsphase mit dem Schließen des Exspirationsventils 22 beendet und es schließt sich ein weiterer Beatmungszyklus an.

Während der Inspirationsphase wird dem Patienten 12 beispielhaft in einem volumenbezogen gesteuerten unterstützenden Beatmungsmodus das für den Patienten 12 eingestellte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuervorrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu kann die Beatmungsvorrichtung 10 mit einem oder mehreren Sensoren datenübertragungsmäßig verbunden sein, welche den Zustand des Patienten oder/und den Betrieb der Beatmungsvorrichtung 10 überwachen. Als einen solchen Sensor weist die Beatmungsvorrichtung 10 einen proximalen Differenzdruck-Durchflusssensor 44 auf, welcher den in der Atemgasleitungsanordnung 30 herrschenden Atemgasfluss, und zwar sowohl den inspiratorischen Atemgasfluss AF wie auch den exspiratorischen Atemgasfluss EF, betragsmäßig erfasst. Der proximale Differenzdruck-Durchflusssensor 44 ist mittels einer Sensorleitungsanordnung 46 mit den Dateneingängen 26 der Steuervorrichtung 18 gekoppelt. Die Sensorleitungsanordnung 46 kann, muss jedoch nicht, elektrische Signalübertragungsleitungen umfassen. Sie kann ebenso Schlauchleitungen aufweisen, die den in Strömungsrichtung beiderseits eines Strömungswiderstands des Differenzdruck-Flusssensors 44 herrschenden Gasdruck an die Dateneingänge 26 übertragen, wo diese von Drucksensoren 27 quantifiziert werden.

Genauer weist die Atemgasleitungsanordnung 30 im bevorzugten Ausführungsbeispiel an ihrem proximalen Längsendbereich 30a einen gesondert ausgebildeten Y-Leitungsabschnitt 47 auf, welcher an seinem distalen Endbereich mit dem zweiten Inspirationsschlauch 36 und dem Exspirationsschlauch 42 verbunden ist und welcher an seinem proximalen Endbereich mit dem proximalen Flusssensor 44 verbunden ist.

Der proximale Flusssensor 44 weist an seinem proximalen Endbereich eine Kopplungsformation 44a auf, mit welcher die Patientenschnittstelle 31, die anstelle eines Tubus auch eine Maske sein könnte, mit dem proximalen Flusssensor 44 und folglich mit der Atemgasleitungsanordnung 30 koppelbar ist.

Der zweite Inspirationsschlauch 36 kann an seinem proximalen Längsendbereich einen proximalen Temperatursensor 48 aufweisen, welcher die Temperatur des Atemgasflusses AF im zweiten Inspirationsschlauch 36 möglichst nah am Patienten 12 misst und an die Steuervorrichtung 18 überträgt.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 50 aufgenommen sein kann.

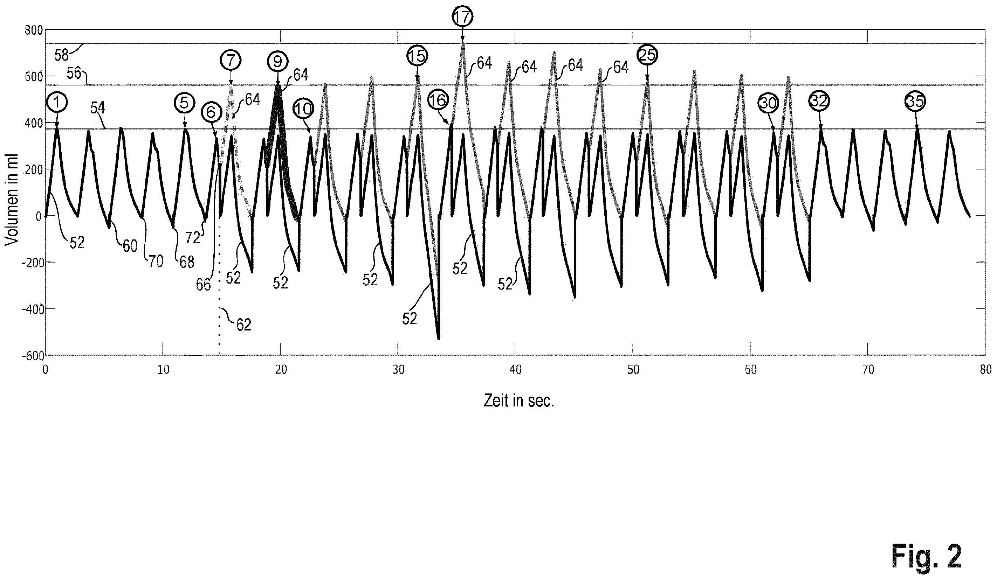

In Figur 2 ist beispielhaft eine graphische Ausgabe der Atemgasmengenbilanz des Patienten 12 über mehrere Atemhübe hinweg gezeigt. Die Abszisse des Koordinatensystems von Figur 2 gibt die Zeit in Sekunden an, die Ordinate die aktuell beim Patienten 12 verbliebene Atemgasmenge, also den Atemgas-Atemtraktinhalt, als Atemgasvolumen in Millilitern.

Ein Graph 52 zeigt das Ergebnis eines herkömmlichen Verfahrens zur Bestimmung einer aktuell beim Patienten 12 als Atemgas-Atemtraktinhalt verbliebenen Atemgasmenge als Atemgasmengenbilanz. Bei der herkömmlichen Bestimmung des Atemgas-Atemtraktinhalts des Patienten 12 wird der Anfangsmengenwert der Atemgasmengenbilanz vor jedem Atemhub von der Steuervorrichtung 18 auf null zurückgesetzt, um eine Drift des Signals 52 zu verhindern. Eine solche Drift könnte durch eine fehlerhafte Kalibration oder/und durch Fertigungstoleranzen des Differenzdruck-Durchflusssensors 44 bewirkt werden. Die Fertigungstoleranzen können sich dahingehend auswirken, dass der Differenzdruck-Durchflusssensor 44 für betragsmäßig ein und denselben Atemgasfluss inspiratorisch und exspiratorisch voneinander betragsmäßig geringfügig abweichende Flusssignale ausgibt bzw. erzeugt.

Die Steuervorrichtung 18 integriert für die Bestimmung des Atemgas-Atemtraktinhalts die vom Differenzdruck-Durchflusssensor 44 erfassten Flüsse über die Dauer eines Atemhubs auf. In der Darstellung von Figur 2 ist jede lokale obere Spitze des Graphen 52 einem anderen Atemhub zugeordnet, wobei die steigende Flanke durch den Inspirationsvorgang eines Atemhubs erzeugt wird und die fallende Flanke durch den Exspirationsvorgang desselben Atemhubs. Da die Atemgasmengenbilanz bzw. der Atemgas-Atemtraktinhalt zu Beginn eines jeden Atemhubs bei herkömmlicher Bestimmung bei null beginnt, werden inspiratorische Atemgasflüsse mit positiven Beträgen und exspiratorische Atemgasflüsse mit negativen Beträgen integriert. Die Atemhübe sind nachfolgend mit fortlaufenden Nummern adressiert, beginnend mit dem Atemhub Nr. 1 bei 0 Sekunden. Zur erleichterten Orientierung sind einige Atemhübe mit ihren Nummern im Kreis identifiziert.

Eine Linie 54 gibt eine Soll-Atemgasmenge bzw. einen Soll-Atemgas-Atemtraktinhalt am Ende einer Inspirationsphase eines jeden Atemhubs an. Dieser Soll-Atemgas-Atemtraktinhalt entspricht dem oben genannten Beatmungs-Tidalvolumen.

Die zur Linie 54 parallele Linie 56 gibt einen ersten Grenzwert eines Atemgas-Atemtraktinhalts an, welcher dem 1,5-Fachen des Sollwerts des Beatmungs-Tidalvolumens entspricht. Die Linie 58 gibt einen zweiten Grenzwert des Atemgas-Atemtraktinhalts an, welche dem Doppelten des Sollwerts des Beatmungs-Tidalvolumens entspricht.

Die in Figur 2 dargestellte Atemgasmengenbilanz ist eine theoretisch erzeugte Atemgasmengenbilanz, zur Veranschaulichung des vorliegend angewandten Verfahrens. Sie ist keine an einem realen Patienten ermittelte Atemgasmengenbilanz.

Bei den Atemhüben Nr. 2 und Nr. 4 von Figur 2 kommt es zu einer übermäßigen Exspiration, bei welcher der Patient 12 mehr Atemgas abgibt als er zuvor während der Inspirationsphase verabreicht bekommen hat. Dies kann beispielsweise durch ein leichtes Husten am Ende der Exspiration oder durch einen spontanen Wegfall eines Strömungshindernisses, etwa einer Schleimansammlung in den Atemwegen, entstehen. Deutlich ist zu erkennen, wie der Folge-Atemhub Nr. 3 in Bezug auf den früheren Atemhub Nr. 2 zu Beginn der Bestimmung des Atemgas-Atemtraktinhalts während des Folge-Atemhubs Nr. 3 von der Steuervorrichtung zwangsweise auf null gesetzt wird, zu erkennen an dem das Ende der Exspiration von Atemhub Nr. 2 mit dem Beginn der Inspiration von Atemhub Nr. 3 verbindenden vertikalen Linienabschnitt 60. Gleiches gilt für den Folge-Atemhub Nr. 5 in Bezug auf seinen unmittelbar vorhergehenden früheren Atemhub Nr. 4.

Die dargestellte übermäßige Exspiration, bei welcher die grafisch dargestellte Atemgasmengenbilanz negativ wird, kann beispielsweise dadurch geschehen, dass für jeden Atemhub die Integration der während seiner Dauer auftretenden Atemgasflüsse bei null beginnt und nicht über das Ende eines Atemhubs hinweg integriert wird. Nicht vergessen werden darf dabei jedoch die Tatsache, dass der Atemhub Nr. 1 zwar bei einer Atemgasmenge von null beginnt, im Atemtrakt 12a des Patienten 12 zu diesem Zeitpunkt jedoch eine von null verschiedene positive Atemgasmenge als anfänglicher Atemgas-Atemtraktinhalt vorhanden war.

Die Beatmungsvorrichtung 10 arbeitet im dargestellten Beispiel in einem volumenbezogen gesteuerten Unterstützungs-Beatmungsmodus, gemäß welchem dem Patienten 12 das eingestellte Beatmung-Tidalvolumen entweder dann verabreicht wird, wenn in der Atemgasleitung der PEEP erfasst wird, was das Ende einer Inspiration anzeigt, oder wenn der Patient 12 Spontanatmung zeigt, also eine Inspirationsanstrengung betreibt bzw. eine Inspiration triggert.

Wie in Fig. 2 gut zu erkennen ist, ist der Exspirationsvorgang in Atemhub Nr. 6 unvollständig und endet durch Triggerung eines Inspirationsvorgangs bei etwa 15 Sekunden (punktierte Linie 62). Aufgrund der eine Signaldrift vermeidenden Konvention des herkömmlichen Verfahrens zur Bestimmung des Atemgas-Atemtraktinhalts des Patienten 12 wird für den bezogen auf den früheren Atemhub Nr. 6 nachfolgenden Folge-Atemhub Nr. 7 der Anfangswert des Atemgas-Atemtraktinhalts bzw. der Atemgasmengenbilanz auf null gesetzt, von wo aus der Inspirationsvorgang des Atemhubs Nr. 7 mit dem Graphen 52 aufgezeichnet wird.

Aufgrund des permanenten Rücksetzens des Atemgas-Atemtraktinhalts des Patienten 12 zu Beginn eines Atemhubs erscheint auch der Atemhub Nr. 7 im Graphen 52 als würde dem Patienten 12 nur ordnungsgemäß das Beatmung-Tidalvolumen zugeführt. Als Konsequenz des tatsächlich mit Atemgas während des Atemhubs Nr. 7 überfüllten Atemtrakts 12a kommt es im Graphen 52 bei der Anwendung des herkömmlichen Verfahrens am Ende des Atemhubs Nr. 7 zu einer drastischen scheinbaren Überentleerung des Atemtrakts 12a des Patienten.

Tatsächlich findet die am Ende von Atemhub Nr. 7 im Graphen 52 dargestellte Überentleerung nicht statt. Die passive Exspiration, die in der Regel nur getrieben ist von dem während der Inspiration erzeugten Überdruck im Atemtrakt des Patienten 12a bezogen auf die Umgebungsatmosphäre als der Senke für exspiratorisches Atemgas, bewirkt lediglich eine Abgabe des aufgrund der vorhergehenden unvollständigen Exspiration im Atemhub Nr. 6 übergroßen Atemgas-Atemtraktinhalts nach Ende des Inspirationsvorgangs von Atemhub Nr. 7.

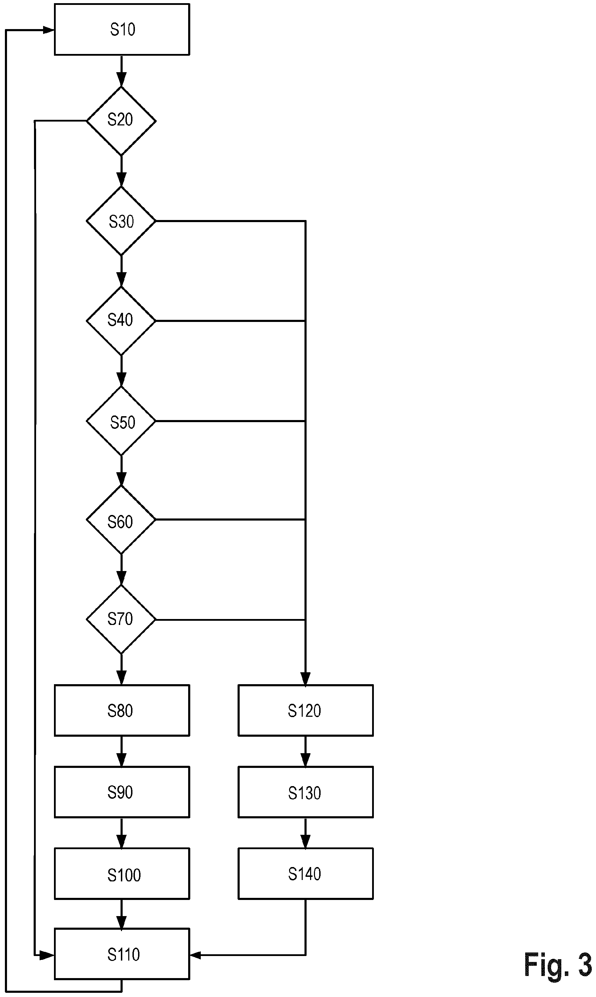

Der Graph 64 in Figur 2 zeigt den nach dem vorliegend beschriebenen erfindungsgemäßen Verfahren bestimmten Atemgas-Atemtraktinhalt. Dieses läuft in der Steuervorrichtung 18 gemäß dem in Figur 3 dargestellten Flussdiagramm ab.

Die Steuervorrichtung 18 fragt mit einer Frequenz f die vom Differenzdruck-Durchflusssensor 44 erfassten Atemgasflusswerte ab. Folglich erhält die Steuervorrichtung 18 jeweils nach Verstreichen einer Zeit $\Delta t = 1/f$ einen neuen Atemgasflusswert.

Bei Schritt S10 in Figur 3 fragt die Steuervorrichtung 18 den Differenzdruck-Durchflusssensor 44 ab und erhält als Antwort einen Atemgasflusswert F.

In dem nachfolgenden Schritt S20 prüft die Steuervorrichtung 18, ob der Atemgasflusswert F zu Beginn eines neuen Atemhubs, also zu Beginn einer neuen Inspirationsphase erfasst wurde, denn nur am Beginn eines neuen Atemhubs ist überhaupt zu prüfen, ob der bisher ermittelte Atemgas-Atemtraktinhalt eines früheren Atemhubs durch entsprechendes Setzen eines Anfangswerts für die Bestimmung des Atemgas-Atemtraktinhalts für den neu beginnenden Folge-Atemhub fortgeschrieben werden soll oder ob die Bestimmung des Atemgas-Atemtraktinhalts bei null beginnen soll.

Dann, wenn der erhaltene Atemgasflusswert F zu Beginn eines Folge-Atemhubs erfasst wurde, schreitet das Verfahren voran zu Schritt S30, in welchem ein Entscheidungswert ermittelt und mit einem Entscheidungs-Schwellenwert verglichen wird.

Der Entscheidungswert ist beispielsweise der auf die inspiratorische Atemgasmenge des vorausgehenden früheren Atemhubs bezogene Unterschiedswert des früheren Atemhubs, welcher einen bilanziellen Unterschied zwischen inspiratorischer und exspiratorischer Atemgasmenge des früheren Atemhubs quantifiziert.

Da der frühere Atemhub Nr. 6 mit einem Anfangswert von 0 ml begonnen hat, ist am Punkt 66 in Figur 2, an welchem die Exspiration im früheren Atemhub Nr. 6 vorzeitig endet und der Folge-Atemhub Nr. 7 beginnt, der Wert der Atemgasmengenbilanz gemäß Graph 52 (und auch gemäß dem an dieser Stelle noch mit dem Graphen 52 deckungsgleichen Graphen 64) der Unterschiedswert zwischen der im früheren Atemhub Nr. 7 inspirierten Atemgasmenge und der im selben Atemhub Nr. 7 exspirierten Atemgasmenge. Der Unterschiedswert beträgt etwa 220 ml. Die inspiratorische Atemgasmenge des früheren Atemhubs Nr. 6 ist der lokale Spitzenwert des Graphen 52, an welchem sich die Steigung des Graphen 52 umgekehrt. Er beträgt etwa 340 ml.

Der Entscheidungswert beträgt daher 220 ml/340 ml = 0,65. Als Entscheidungs-Schwellenwert wurde aufgrund von vorhergehenden Experimenten und Untersuchungen ein Wert von 0,25 gewählt. Der Entscheidungswert übersteigt den Entscheidungs-Schwellenwert, sodass das Verfahren weiter zu Schritt S40 schreitet.

In Schritt S40 wird überprüft, ob eine inkrementelle Zählvariable, mit welcher die Häufigkeit eines unmittelbar aufeinander folgenden Auftretens von Überschreitungen des Entscheidungs-Schwellenwerts durch den Entscheidungswert gezählt wird, kleiner als oder gleich wie ein vorbestimmter Grenzwert ist, welcher eine maximal zulässige Zahl unmittelbar aufeinander folgender Überschreitungen des Entscheidungs-Schwellenwerts angibt. Im vorliegenden Fall beträgt der vorbestimmte Grenzwert 3.

Da in den früheren Atemhüben Nrn. 1 bis 6 bisher der Entscheidungs-Schwellenwert wegen der im Wesentlichen vollständigen Exspiration nicht überschritten wurde, sondern dies das erste Mal zu Beginn des Folge-Atemhubs Nr. 7 geschieht, schreitet das Verfahren voran zu Schritt S50.

In Schritt S50 wird überprüft, ob die oben angegebene Gleichung 1 für die letzten 10 Atemhübe nicht öfter als 50 % erfüllt war, wobei für den Quotienten-Schwellenwert aufgrund von Experimenten und medizinischen Untersuchungen 0,2 gewählt ist.

Da bis zum Beginn des Folge-Atemhubs Nr. 7 keine vorangehenden 10 Atemhübe existieren, wird der Schritt S50 für die bestehende Anzahl an vorangehenden 6 Atemhüben ausgeführt.

Die Verwendung von Gleichung 1 wird nachfolgend am Beispiel des Atemhubs Nr. 4 mit i=4 erläutert: das endexspiratorische Atemgasvolumen des Atemhubs Nr. 4, siehe Punkt 68, beträgt etwa -30 ml. Das endexspiratorische Atemgasvolumen des vorhergehenden Atemhubs Nr. i-1=3, siehe Punkt 70, beträgt etwa 0 ml. Das endinspiratorische Atemgasvolumen des Atemhubs Nr. 4 liegt knapp unterhalb des Soll-Beatmungs-Tidalvolumens bei etwa 350 ml. Mit Gleichung 1 ergibt sich aus diesen Werten für die linke Seite der Gleichung 1 der Wert -0,08, welcher schon alleine wegen des negativen Vorzeichens sicher nicht größer als der Quotienten-Schwellenwert von 0,2 ist. Für die übrigen Atemhübe der Nrn. 1 bis 6 sieht das Ergebnis ähnlich aus, der Quotienten-Schwellenwert wurde nicht überschritten. Folglich war Gleichung 1 in den dem Folge-Atemhub Nr. 7 vorangehenden Atemhüben für weniger als die Hälfte der Atemhübe erfüllt, wodurch der beispielhaft einen Wert von 50 % betragende Häufigkeits-Schwellenwert nicht erreicht wurde. Das Verfahren schreitet dann voran zu Schritt S60.

In Schritt S60 wird geprüft, ob im früheren Atemhub Nr. 5 im Verhältnis zum unmittelbar vorhergehenden Atemhub Nr. 6 eine übermäßig große Exspiration stattfand. Eine große Exspiration, bei welcher mehr Atemgas vom Patienten 12 abgegeben wird als zuvor inspiriert wurde, führt zu einem negativen endexspiratorischen Atemgasvolumen. Das Kriterium dafür, dass keine übermäßig große Exspiration vorliegt, ist, ob das endexspiratorische Atemgasvolumen des unmittelbar vorhergehenden früheren Atemhubs Nr. 6 größer ist als das negative endexspiratorische Atemgasvolumen des früheren Atemhubs Nr. 5 (s. Gleichung 2).

$$V_{endexp,i} > -V_{endexp,i-1} \hspace{4cm} \text{Gl. 2}$$

Das endexspiratorische Atemgasvolumen des Atemhubs Nr. 5 ist zwar negativ, aber mit etwa -25 ml nur geringfügig. Das endexspiratorische Atemgasvolumen des Atemhubs Nr. 6 mit etwa 220 ml ist größer als das mit -1 multiplizierte endexspiratorische Atemgasvolumen des Atemhubs Nr. 5. Daher schreitet das Verfahren voran zu Schritt S70.

In Schritt S70 wird überprüft, ob in einer vorbestimmten Zeitspanne vor dem Folge-Atemhub Nr. 7, etwa während der vorhergehenden letzten beiden Atemhübe Nr. 5 und Nr. 6, der PEEP erhöht wurde, was nicht der Fall ist. Folglich schreitet das Verfahren voran zu Schritt S80.

Wenn Schritt S80 erreicht wird, ist klar, dass alle Kriterien für das Erkennen einer unvollständigen Exspiration und damit einer Atemstapelung erfüllt sind. In Schritt S80 wird der Beatmungsmodus von der bisher ausgeführten volumenbezogen gesteuerten unterstützten Beatmung auf eine druckbezogen gesteuerte unterstützte Beatmung geändert. Weiterhin wird als Druck-Unterstützungswert für den geänderten Beatmungsmodus bevorzugt ein Druckwert gesetzt, welcher dem durchschnittlichen endinspiratorischen Atemgasdruck einer vorbestimmten Anzahl unmittelbar zurückliegender Atemhübe, etwa der letzten 10 Atemhübe, abzüglich des PEEP eingestellt wird. Hierdurch soll nach einer erkannten unvollständigen Exspiration das erneute Verabreichen des Tidalvolumens verhindert werden. Das Verfahren schreitet dann voran zu Schritt S90.

Der Schritt S80 einer Änderung des Beatmungsmodus ist nur eine mögliche Variante des Verfahrens. Häufig ist von behandelnden Therapeuten das selbstständige ändern des Beatmungsmodus durch die Beatmungsvorrichtung nicht erwünscht. Das Verfahren kann auch unmittelbar von Schritt S70 zu Schritt S90 weitergehen.

In dem graphisch in Figur 2 dargestellten Beatmungsvorgang hat kein Wechsel des Beatmungsmodus stattgefunden.

In Schritt S90 wird die Zählvariable, welche die Häufigkeit unmittelbar aufeinanderfolgend auftretender unvollständiger Exspiration anzeigt, um 1 erhöht. Das Verfahren schreitet dann voran zu Schritt S100.

In Schritt S100 wird das endexspiratorische Atemgasvolumen des unmittelbar vorhergehenden früheren Atemhubs Nr. 6 als Kontinuitäts-Anfangswert für die Bestimmung des Atemgas-Atemtraktinhalts während des Folge-Atemhubs Nr. 7 gesetzt. Das Verfahren schreitet voran zu Schritt S110.

In Schritt S110 wird durch numerische Integration aus dem in Schritt S10 erhaltenen Atemgasfluss das bisher geströmte Atemgasvolumen ermittelt. Dabei wird davon ausgegangen, dass bis zur Erfassung des nächsten Atemgasflusses, also für die Dauer $\Delta t$, der erfasste Atemgasfluss F strömt. Der in Schritt S 110 ermittelte Atemgas-Atemtraktinhalt AL für den k-ten erfassten Atemgasfluss aus insgesamt n Atemgasflüssen des betrachteten Folge-Atemhubs entspricht also der folgenden Gleichung 3:

$$AL(k) = V_{Anfangswert} + \sum_{k=1}^{n} F(k) \cdot \Delta t \qquad\qquad \text{Gl. 3}$$

wobei $V_{Anfangswert}$ der Anfangswert für die Bestimmung des Atemgas-Atemtraktinhalts im Folge-Atemhub ist. Wie oben beschrieben wurde, ist nach Durchlaufen des den Schritt S100 enthaltenden Verfahrensastes der Anfangswert der Kontinuitäts-Anfangswert, d. h. das endexspiratorische Atemgasvolumen im Atemtrakt des Patienten in dem dem Folge-Atemhub unmittelbar vorausgehenden früheren Atemhub.

Nach Ausführung der numerischen Integration in Schritt S110 kehrt das Verfahren zurück zu Schritt S10, wo der nächste Atemgasfluss erfasst wird.

Da das Verfahren nur zur Bestimmung eines Anfangswerts für eine Bestimmung eines Atemgas-Atemtraktinhalt des Patienten 12 dient, schreitet das Verfahren bei Schritt S20 danach, wenn der erfasste Atemgasfluss nicht am Anfang eines Atemhubs steht, unmittelbar zur numerischen Integration des Schritts S110.

Die Schritte S40 bis S70 müssen nicht ausgeführt werden, erhöhen jedoch die Genauigkeit des Verfahrens. Das Verfahren kann auch ausschließlich in Schritt S30 anhand der Bildung des Entscheidungswerts und dessen Vergleichs mit einem Entscheidungs-Schwellenwert zu den Verlaufsästen mit den Schritten S100 oder S140 führen.

Dann, wenn in einem der Schritte S30 bis S70 eines der genannten Kriterien nicht wie oben beschrieben beurteilt wird, erreicht der Verfahrensablauf nicht die Schritte S80 oder S90, sondern gelangt in den alternativen Ast, wo als nächstes der Schritt S120 abgearbeitet wird.

Der Schritt S120, der nicht zwingend vorhanden sein muss, bestätigt die Fortsetzung des bisher gewählten volumenbezogen gesteuerten, unterstützenden Beatmungsmodus.

Auf jeden Fall wird bei abweichender Kriterienbeurteilung in einem der Schritte S30 bis S70 der mit dem zuvor erläuterten Schritt S90 korrespondierende alternative Schritt S130 erreicht, in welchem die Zählvariable zur Zählung der Häufigkeit unmittelbar aufeinanderfolgender Atemstapelungen, also unvollständige Exspiration, auf null zurückgesetzt wird, da der unmittelbar vorhergehende frühere Atemhub keine Atemstapelung aufwies.

Das Verfahren fährt dann fort zu Schritt S140, in welchem der Anfangswert für die Bestimmung des Atemgas-Atemtraktinhalts für den Folge-Atemhub auf den Rücksetz-Anfangswert null zurückgesetzt wird. Dies bedeutet, dass dann, wenn der Verfahrensast mit dem Schritt S140 durchlaufen wird, $V_{Anfangswert}$ gleich null ist.

Anschließend schreitet das Verfahren voran zu dem bereits oben erläuterten Schritt S110, in welchem die numerische Integration des erfassten Artenflusses F(k) erfolgt.

Zur besseren Übersichtlichkeit sind in Figur 2 der Graph 52, welcher herkömmlich den Atemgas-Atemtraktinhalt des Patienten 12 mit Rücksetzen des Anfangswerts auf null vor jedem Atemhub zeigt, und der Graph 64, welcher die Ermittlung des Atemgas-Atemtraktinhalts des Patienten 12 gemäß dem vorliegend vorgestellten Verfahren zeigt, mehrfach mit Bezugszeichen versehen. Grundsätzlich gilt, dass in Figur 2 die die Nulllinie stark unterschreitenden Graphenabschnitte zum Graphen 52 gehören und die die Linie 56 annähernd oder vollständig erreichenden Spitzenwerte zum Graphen 64 gehören. Zwischen einer Einmündung des Graphen 64 in den Graphen 52 und einer erneuten Trennung des Graphen 64 vom Graphen 52 verlaufen die beiden Graphen 64 und 52 längs einer gemeinsamen Linie, was sich durch die gemeinsame Integrationsvorschrift erklärt.

Um einem die graphische Ausgabe des Atemgas-Atemtraktinhalts beobachtenden Therapeuten die unvollständige Exspiration und das daran anschließende erneute Verabreichen des Tidalvolumens möglichst leicht, schnell und sicher erkennbar zu machen, ist die Steuervorrichtung 18 dazu ausgebildet, die graphische Darstellung des Atemgas-Atemtraktinhalts als Funktion der Zeit ab dem Zeitpunkt zu verändern, in welchem der Kontinuitäts-Anfangswert als der Atemtraktinhalt-Anfangswert gesetzt wird. Diese Änderung der Darstellung kann beispielsweise bis zu einem Erreichen eines endexspiratorischen Atemtraktinhalts fortdauern, welcher einen vorbestimmten Grafikänderungs-Schwellenwert unterschreitet oder sich betragsmäßig nicht mehr als ein vorbestimmter Grafikänderungs-Betrag von dem durchschnittlichen endexspiratorischen Atemtraktinhalts der letzten n Atemhübe unterscheidet, wobei n beispielsweise 5 oder 10 oder eine andere ganze Zahl sein kann.

Für den Atemhub Nr. 7 ist beispielhaft die graphische Ausgabe des Atemgas-Atemtraktinhalts durch Änderung der Linienart und der Linienfarbe geändert. Für den Atemhub Nr. 9 ist beispielhaft die graphische Ausgabe des Atemgas-Atemtraktinhalts durch Änderung der Linienstärke von dünner nach dicker geändert.

Beim Atemhub Nr. 7, nach der unvollständigen Exspiration des Atemhubs Nr. 6, unterscheiden sich die Ergebnisse der herkömmlichen Bestimmung des Atemgas-Atemtraktinhalts und die Ergebnisse der Bestimmung gemäß dem vorliegend diskutierten Verfahren erstmals. Es folgt dann ein Atemhub Nr. 8 mit wiederum unvollständiger Exspiration, ein normaler Atemhub Nr. 9, ein Atemhub Nr. 10 mit unvollständiger Exspiration, worauf hin stets Atemhübe mit gerader Nummer bis zu Atemhub Nr. 30 folgen, welche eine unvollständige Exspiration aufweisen, auf welche hin Atemhübe mit ungeradzahliger Nummer und vollständiger Espiration folgen. Ab Atemhub Nr. 32, einschließlich dieses Atemhubs, treten keine Atemstapelungen mehr auf.

Hinzuweisen ist auf folgende Besonderheiten: am Ende der Exspiration des Atemhubs 15 atmet der Patient 12 tief aus, sodass die endexspiratorische Atemgasmenge im Atemtrakt des Patienten 12a negativ wird. Da die endinspiratorische Atemgasmenge des Atemhubs 15 positiv ist, wie im Übrigen jede endinspiratorische Atemgasmenge, ist der in Schritt S30 ermittelte Entscheidungswert ebenfalls negativ und somit kleiner als der Entscheidungs-Schwellenwert. Als Folge wird der Rücksetz-Anfangswert als der Atemtraktinhalt-Anfangswert für die Bestimmung des Atemgas-Atemtraktinhalts des Folge-Atemhubs Nr. 16 gesetzt.

In Atemhub Nr. 16 triggert der Patient 12 unmittelbar nach Ende der Inspiration eine weitere Inspirationsphase, sodass dem Patienten 12 unmittelbar im Anschluss an ein erstes Tidalvolumen ein zweites Tidalvolumen verabreicht wird. Nach der anschließenden Exspiration im Atemhub Nr. 17 ist der in Schritt S30 gebildete Entscheidungswert wegen der hohen endinspiratorischen Atemgasmenge des Atemhubs Nr. 17 kleiner als der Entscheidungs-Schwellenwert, sodass für den nachfolgenden Folge-Atemhub Nr. 18 der Rücksetz-Anfangswert als der Atemtraktinhalt-Anfangswert gesetzt wird.

Die graphische Darstellung des Beatmungsvorgangs durch Anzeige des Atemgas-Atemtraktinhalts als Funktion der Zeit in Figur 2 mit dem Graphen 64 vermeidet eine unerwünschte Drift und gibt dennoch intuitiv besser nachvollziehbare Information über die inspiratorischen und exspiratorischen Atemgasvolumina des Beatmungsvorgangs.

## Patentansprüche

1. Verfahren zur Bestimmung eines Atemtraktinhalts an Atemgas, welcher nach mehreren unter Beteiligung einer Beatmungsvorrichtung (10) ausgeführten Atemhüben im Atemtrakt (12a) eines wenigstens teilweise künstlich beatmeten Patienten (12) vorhanden ist, wobei die mehreren Atemhübe wenigstens eine frühere Atemhubmenge und wenigstens einen auf die frühere Atemhubmenge folgenden Folge-Atemhub aufweisen, wobei die frühere Atemhubmenge einen früheren Atemhub oder eine Mehrzahl von aufeinander folgenden früheren Atemhüben aufweist, wobei das Verfahren ein quantitatives Erfassen von inspiratorischen und exspiratorischen Atemgasflüssen (S10) durch wenigstens einen Atemgas-Flusssensor (44) und eine Summation von erfassten inspiratorischen und exspiratorischen Flusswerten zu dem Atemgas-Atemtraktinhalt (S110) umfasst,
**dadurch gekennzeichnet, dass** ein Atemtraktinhalt-Anfangswert, mit welchem die Bestimmung des Atemtraktinhalts an Atemgas für den Folge-Atemhub beginnt, abhängig von einem Atemgasmengen-Unterschied zwischen einer während der früheren Atemhubmenge dem Patienten (12) zugeführten inspiratorischen Atemgasmenge und einer während der früheren Atemhubmenge vom Patienten (12) abgegebenen exspiratorischen Atemgasmenge auf einen näher bei null als bei einem den Atemgasmengen-Unterschied quantitativ angebenden Unterschiedswert gelegenen Rücksetz-Anfangswert (S140) oder auf einen näher beim Unterschiedswert als bei null gelegenen Kontinuitäts-Anfangswert gesetzt wird (S100).

2. Verfahren nach Anspruch 1,
**dadurch gekennzeichnet, dass** der Rücksetz-Anfangswert null ist oder/und dass der Kontinuitäts-Anfangswert der Unterschiedswert ist.

3. Verfahren nach einem der Ansprüche 1 oder 2,
**dadurch gekennzeichnet, dass** der Atemtraktinhalt-Anfangswert während einer wenigstens teilweise künstlichen Beatmung eines Patienten (12) wiederholt bestimmt wird.

4. Verfahren nach einem der vorhergehenden Ansprüche,
**dadurch gekennzeichnet, dass** das Verfahren eine Ermittlung eines Entscheidungswerts (S30) umfasst, wobei der Entscheidungswert den Atemgasmengen-Unterschied, insbesondere den Unterschiedswert, repräsentiert, wobei abhängig vom Entscheidungswert der Rücksetz-Anfangswert oder der Kontinuitäts-Anfangswert als der Atemtraktinhalt-Anfangswert für den Folge-Atemhub bestimmt wird.

5. Verfahren nach Anspruch 4,
**dadurch gekennzeichnet, dass** der Entscheidungswert proportional zu einem Quotienten aus dem Unterschiedswert und einer dem Patienten unter Beteiligung der Beatmungsvorrichtung (10) während der früheren Atemhubmenge zugeführten inspiratorischen Atemgasmenge ist (S30).

6. Verfahren nach Anspruch 4 oder 5,
**dadurch gekennzeichnet, dass** die Bestimmung des Atemtraktinhalt-Anfangswerts abhängig vom Entscheidungswert einen Vergleich des Entscheidungswerts mit einem vorbestimmten Entscheidungs-Schwellenwert umfasst, wobei der Atemtraktinhalt-Anfangswert abhängig von einem Ergebnis des Vergleichs bestimmt wird (S30).

7. Verfahren nach einem der vorhergehenden Ansprüche, unter Einbeziehung des Anspruchs 3,
**dadurch gekennzeichnet, dass** der Rücksetz-Anfangswert dann unabhängig vom Atemgasmengen-Unterschied als der Atemtraktinhalt-Anfangswert bestimmt wird, wenn zuvor bei einer vorbestimmten ersten Anzahl von aufeinander folgenden Bestimmungen des Atemtraktinhalt-Anfangswerts jedes Mal der Kontinuitäts-Anfangswert als der Atemtraktinhalt-Anfangswert bestimmt wurde (S40).

8. Verfahren nach einem der vorhergehenden Ansprüche, unter Einbeziehung des Anspruchs 3,
**dadurch gekennzeichnet, dass** der Rücksetz-Anfangswert dann unabhängig vom Atemgasmengen-Unterschied als der Atemtraktinhalt-Anfangswert bestimmt wird, wenn zuvor innerhalb einer vorbestimmten zweiten Anzahl von Bestimmungen des Atemtraktinhalt-Anfangswerts die Anzahl von Bestimmungen, die den Kontinuitäts-Anfangswert zum Ergebnis hatten, einen vorbestimmten Anteils-Schwellenwert erreicht oder übersteigt, wobei der Anteils-Schwellenwert kleiner als die zweite Anzahl ist.

9. Verfahren nach einem der vorhergehenden Ansprüche, unter Einbeziehung des Anspruchs 3,
**dadurch gekennzeichnet, dass** der Rücksetz-Anfangswert dann unabhängig vom Atemgasmengen-Unterschied als der Atemtraktinhalt-Anfangswert bestimmt wird, wenn zuvor innerhalb einer vorbestimmten Anzahl von Atemhüben gilt, dass die Häufigkeit, mit welcher ein Quotient aus einem ersten Atemtraktinhalt-Unterschied und einem vom ersten verschiedenen zweiten Atemtraktinhalt-Unterschied einen vorbestimmten Quotienten-Schwellenwert erreicht oder übersteigt, einen Häufigkeits-Schwellenwert erreicht oder übersteigt, wobei bevorzugt der erste Atemtraktinhalt-Unterschied ein Unterschied zwischen dem Atemtraktinhalt an Atemgas am Ende eines bestimmten Atemhubs und dem Atemtraktinhalt an Atemgas am Ende einer dem bestimmten Atemhub vorausgehenden früheren Atemhubmenge ist oder/und wobei der zweite Atemtraktinhalt-Unterschied ein Unterschied zwischen einem Atemtraktinhalt an Atemgas am Ende einer Inspirationsphase des bestimmten Atemhubs und dem Atemtraktinhalt an Atemgas am Ende der dem bestimmten Atemhub vorausgehenden früheren Atemhubmenge ist (S50).

10. Verfahren nach einem der vorhergehenden Ansprüche,
**dadurch gekennzeichnet, dass** der Rücksetz-Anfangswert dann unabhängig vom Atemgasmengen-Unterschied als der Atemtraktinhalt-Anfangswert bestimmt wird, wenn für wenigstens eine der beiden dem aktuellen Folge-Atemhub, für welchen der Atemtraktinhalt-Anfangswert bestimmt wird, unmittelbar vorausgehenden früheren Atemhubmengen ermittelt wird, dass ein aus den erfassten exspiratorischen Atemgasflüssen ermitteltes exspiratorisches Atemgasvolumen der früheren Atemhubmenge betragsmäßig größer ist als ein aus den inspiratorischen Atemgasflüssen ermitteltes inspiratorisches Atemgasvolumen derselben früheren Atemhubmenge.

11. Verfahren nach einem der vorhergehenden Ansprüche,
**dadurch gekennzeichnet, dass** der Rücksetz-Anfangswert dann unabhängig vom Atemgasmengen-Unterschied als der Atemtraktinhalt-Anfangswert bestimmt wird, wenn innerhalb einer vorbestimmten Zeitspanne vor der aktuellen Bestimmung des Atemtraktinhalt-Anfangswerts eine quantitative Änderung des PEEP erfasst wurde (S70).

12. Verfahren nach einem der vorhergehenden Ansprüche,
**dadurch gekennzeichnet, dass** der Atemtraktinhalt an Atemgas graphisch als Funktion der Zeit an einer Ausgabevorrichtung (28) ausgegeben wird, wobei insbesondere dann, wenn der Kontinuitäts-Anfangswert als der Atemtraktinhalt-Anfangswert gesetzt wird, die graphische Ausgabe des Atemtraktinhalts als Funktion der Zeit verändert wird.

13. Beatmungsvorrichtung (10) zur wenigstens teilweise künstlichen Beatmung eines Patienten (12), umfassend:

    - eine Atemgas-Quellenanordnung (15), welche ein inspiratorisches Atemgas zur künstlichen Beatmung des Patienten (12) bereitstellt,
    - eine Strömungsveränderungsvorrichtung (16), welche dazu ausgebildet ist, einen inspiratorischen Atemgasfluss (AF) zu erzeugen und betragsmäßig zu verändern,

- eine Atemgasleitungsanordnung (30) mit einem im Betrieb dem Patienten (12) näher gelegenen proximalen Längsende und mit einem im Betrieb vom Patienten (12) weiter entfernt gelegenen distalen Längsende, um den inspiratorischen Atemgasfluss (AF) von der Atemgas-Quellenanordnung (15) zum Patienten (12) hin zu fördern,
- eine Flusssensoranordnung (44), welche dazu ausgebildet ist, den inspiratorischen Atemgasfluss (AF) ebenso wie einen exspiratorischen Atemgasfluss (EF) betragsmäßig zu erfassen,
- eine Steuervorrichtung (18) mit einem Datenspeicher (19), wobei die Steuervorrichtung (18) signalübertragungsmäßig mit dem Datenspeicher (19) und mit der Flusssensoranordnung (44) verbunden ist und welche dazu ausgebildet ist, die Betriebsleistung der Strömungsveränderungsvorrichtung (16) zur Veränderung des inspiratorischen Atemgasflusses (AF) zu steuern,

**dadurch gekennzeichnet, dass** die Steuervorrichtung (16) dazu ausgebildet ist, das Verfahren nach einem der vorhergehenden Ansprüche auszuführen, wobei bevorzugt die Beatmungsvorrichtung (10) eine graphische Ausgabevorrichtung (28) aufweist und die Steuervorrichtung (28) dazu ausgebildet ist, das Verfahren nach Anspruch 12 auszuführen.

14. Beatmungsvorrichtung (10) nach Anspruch 13,
**dadurch gekennzeichnet, dass** die Beatmungsvorrichtung (10) dazu ausgebildet ist, eine künstliche Beatmung in einem Unterstützungs-Beatmungsmodus durchzuführen, in welchem die Steuervorrichtung (16) eine Inspirationsanstrengung des beatmeten Patienten (12) erfasst und auf die Erfassung der Inspirationsanstrengung hin die Strömungsveränderungsvorrichtung (16) ansteuert, dem Patienten (12) eine inspiratorische Atemgasmenge über die Atemgasleitungsanordnung (30) zu verabreichen, wobei bevorzugt die Steuervorrichtung (18) dazu ausgebildet ist, auf Grundlage des bestimmten Atemtraktinhalt-Anfangswerts oder/und auf Grundlage des bestimmten Atemtraktinhalts an Atemgas, eine unvollständige Exspiration zu erkennen, und dann, wenn die Steuervorrichtung (18) eine unvollständige Exspiration erkannt hat, den Beatmungsmodus zu ändern, insbesondere zwischen einem volumenbezogen gesteuerten Beatmungsmodus und einem druckbezogen gesteuerten Beatmungsmodus zu wechseln.

15. Beatmungsvorrichtung (10) nach einem der Ansprüche 13 bis 14,
**dadurch gekennzeichnet, dass** die Flusssensoranordnung (44), auf Grundlage von deren Erfassungswerten der Atemtraktinhalt-Anfangswert bestimmt wird, eine proximale Flusssensoranordnung (44) ist.

## Claims

1. Method for determining respiratory gas content in a respiratory tract, which is present in the respiratory tract (12a) of an at least partially artificially ventilated patient (12) after several breaths performed under the participation of a ventilation apparatus (10), where the several breaths exhibit at least one earlier breath set and at least one subsequent breath following the earlier breath set, where the earlier breath set exhibits one earlier breath or a plurality of successive earlier breaths, where the method comprises quantitative acquisition of inspiratory and expiratory respiratory gas flows (S10) through at least one respiratory gas flow sensor (44) and summation of acquired inspiratory and expiratory flow values to the respiratory tract content of respiratory gas (S110),
**characterized in that** a respiratory tract content starting value, with which the determination of the respiratory gas content in a respiratory tract for the subsequent breath begins, depending on a respiratory gas quantity difference between an inspiratory respiratory gas quantity supplied to the patient (12) during the earlier breath set and an expiratory respiratory gas quantity exhaled by the patient (12) during the earlier breath set, is set to a reset starting value lying nearer to zero than to a difference value indicating quantitatively the respiratory gas quantity difference (S140) or to a continuity starting value lying nearer to the difference value than to zero (S100).

2. Method according to Claim 1,
**characterized in that** the reset starting value is zero and/or that the continuity starting value is the difference value.

3. Method according to one of the Claims 1 or 2,
**characterized in that** the respiratory tract content starting value is determined repeatedly during an at least partial artificial ventilation of a patient (12).

4. Method according to one of the preceding Claims,
**characterized in that** the method comprises ascertaining a decision value (S30), where the decision value represents the respiratory gas quantity difference, in particular the difference value, where depending on the decision value the reset starting value or the continuity starting value is determined as the respiratory tract content starting value for the subsequent breath.

5. Method according to Claim 4,
   **characterized in that** the decision value is proportional to a ratio of the difference value and an inspiratory respiratory gas quantity supplied to the patient under the participation of the ventilation apparatus (10) during the earlier breath set (S30).

6. Method according to Claim 4 or 5,
   **characterized in that** the determination of the respiratory tract content starting values comprises, in dependence on the decision value, a comparison of the decision value with a predetermined decision threshold value, where the respiratory tract content starting value is determined in dependence of a result of the comparison (S30).

7. Method according to one of the preceding Claims, in consideration of Claim 3,
   **characterized in that** the reset starting value is determined independently from the respiratory gas quantity difference as the respiratory tract content starting value when previously in a predetermined first number of successive determinations of the respiratory tract content starting value each time the continuity starting value was determined as the respiratory tract content starting value (S40).

8. Method according to one of the preceding Claims, in consideration of Claim 3,
   **characterized in that** the reset starting value is determined independently from the respiratory gas quantity difference as the respiratory tract content starting value when previously within a predetermined second number of determinations of the respiratory tract content starting value the number of determinations which had as a result the continuity starting value reaches or exceeds a predetermined fraction threshold value, where the fraction threshold value is smaller than the second number.

9. Method according to one of the preceding Claims, in consideration of Claim 3,
   **characterized in that** the reset starting value is determined as the respiratory tract content starting value independently from the respiratory gas quantity difference when previously within a predetermined number of breaths it is the case that the frequency at which a ratio of a first respiratory tract content difference and a second respiratory tract content difference which differs from the first reaches or exceeds a predetermined ratio threshold value, reaches or exceeds a frequency threshold value, where preferably the first respiratory tract content difference is a difference between the respiratory gas content in a respiratory tract at the end of a particular breath and the respiratory gas content in a respiratory tract at the end of an earlier breath set preceding the particular breath and/or where the second respiratory tract content difference is a difference between a respiratory gas content in a respiratory tract at the end of an inspiration phase of the determined breath and the respiratory gas content in a respiratory tract at the end of the earlier breath set preceding the particular breath (S50).

10. Method according to one of the preceding Claims,
    **characterized in that** the reset starting value is determined as the respiratory tract content starting value independently from the respiratory gas quantity difference when for at least one of the two earlier breath sets directly preceding the current subsequent breath for which the respiratory tract content starting value is being determined, it is ascertained that an expiratory respiratory gas volume of the earlier breath set ascertained from the acquired expiratory respiratory gas flows is quantitatively greater than an inspiratory respiratory gas volume of the same earlier breath set ascertained from the inspiratory respiratory gas flows.

11. Method according to one of the preceding Claims,
    **characterized in that** the reset starting value is determined as the respiratory tract content starting value independently from the respiratory gas quantity difference when within a predetermined time interval before the current determination of the respiratory tract content starting value a quantitative change in the PEEP was acquired (S70).

12. Method according to one of the preceding Claims,

    **characterized in that** the respiratory gas content in a respiratory tract is output graphically as a function of time at an output device (28),
    wherein in particular, when the continuity starting value is set as the respiratory tract content starting value, the graphic output of the respiratory tract content is changed as a function of time.

**13.** Ventilation apparatus (10) for at least partial artificial ventilation of a patient (12), comprising:

- a respiratory gas source arrangement (15) which provides an inspiratory respiratory gas for artificial ventilation of the patient (12),
- a flow modification device (16) which is configured create and quantitatively to modify an inspiratory respiratory gas flow (AF),
- a respiratory gas line arrangement (30) with a proximal longitudinal end which during operation lies nearer to the patient (12) and with a distal longitudinal end which during operation lies further away from the patient (12), in order to convey the inspiratory respiratory gas flow (AF) from the respiratory gas source arrangement (15) up to the patient (12),
- a flow sensor arrangement (44) which is configured quantitatively to acquire the inspiratory respiratory gas flow (AF) and likewise an expiratory respiratory gas flow (EF),
- a control device (18) with a data store (19), where the control device (18) is linked for signal transmission with the data store (19) and with the flow sensor arrangement (44) and which is configured to control the operational performance of the flow modification device (16) for modifying the inspiratory respiratory gas flow (AF), **characterized in that** the control device (16) is configured to implement the method according to one of the preceding Claims, wherein preferably the ventilation apparatus (10) exhibits a graphic output device (28), and the control device (28) is configured to implement the method according to Claim 12.

**14.** Ventilation apparatus (10) according to Claim 13, **Characterized in that** the ventilation apparatus (10) is configured to perform artificial ventilation in a support ventilation mode, in which the control device (16) acquires an inspiration effort of the ventilated patient (12) and on acquiring the inspiration effort actuates the flow modification device (16) to administer to the patient (12) an inspiratory respiratory gas quantity via the respiratory gas line arrangement (30), wherein preferably the control device (18) is configured to recognize an incomplete expiration on the basis of the determined respiratory tract content starting value and/or on the basis of the determined respiratory tract content of respiratory gas, and when the control device (18) has recognized an incomplete expiration, to change the ventilation mode, in particular to change between a volume-relatedly controlled ventilation mode and a pressure-relatedly controlled ventilation mode.

**15.** Ventilation apparatus (10) according to one of the Claims 13 to 14, **Characterized in that** the flow sensor arrangement (44) on the basis of whose acquisition values the respiratory tract content starting value is determined, is a proximal flow sensor arrangement (44).

**Revendications**

**1.** Procédé de détermination d'un contenu de tractus respiratoire en gaz respiratoire qui est présent dans le tractus respiratoire (12a) d'un patient (12) au moins partiellement ventilé artificiellement après plusieurs courses respiratoires effectuées avec la participation d'un dispositif de ventilation (10), la pluralité de courses respiratoires présentant au moins une quantité de course respiratoire antérieure et au moins une course respiratoire subséquente suivant la quantité de course respiratoire antérieure, la course respiratoire antérieure comprenant une course respiratoire antérieure ou une pluralité de courses respiratoires antérieures successives, le procédé comprenant une détection quantitative de flux de gaz respiratoire inspiratoire et expiratoire (S10) par au moins un capteur de flux de gaz respiratoire (44) et une sommation de valeurs de flux inspiratoire et expiratoire détectées au contenu de gaz respiratoire expiratoire (S110), **caractérisé en ce qu'**une valeur initiale de contenu de tractus respiratoire, avec laquelle commence la détermination du contenu de tractus respiratoire en gaz respiratoire pour la course respiratoire subséquente, en fonction d'une différence de quantité de gaz respiratoire entre une quantité de gaz respiratoire inspiratoire fournie au patient (12) pendant la quantité de course respiratoire antérieure et une quantité de gaz respiratoire expiratoire délivrée par le patient (12) pendant la quantité de course respiratoire antérieure, est fixée à une valeur initiale de réinitialisation (S140) plus proche de zéro que d'une valeur de différence indiquant quantitativement la différence de quantité de gaz respiratoire, ou à une valeur initiale de continuité plus proche de la valeur de différence que de zéro (S100).

**2.** Procédé selon la revendication 1, **caractérisé en ce que** la valeur initiale de réinitialisation est nulle ou/et **en ce que** la valeur initiale de continuité est la valeur de différence.

**3.** Procédé selon l'une des revendications 1 ou 2,
**caractérisé en ce que** la valeur initiale du contenu de tractus respiratoire est déterminée de manière répétée pendant une ventilation au moins partiellement artificielle d'un patient (12).

**4.** Procédé selon l'une des revendications précédentes,
**caractérisé en ce que** le procédé comprend une détermination d'une valeur de décision (S30), la valeur de décision représentant la différence de quantité de gaz respiratoire, en particulier la valeur de différence, dans lequel, en fonction de la valeur de décision, la valeur initiale de réinitialisation ou la valeur initiale de continuité est déterminée comme étant la valeur initiale de contenu de tractus respiratoire pour la course respiratoire subséquente.

**5.** Procédé selon la revendication 4,
**caractérisé en ce que** la valeur de décision est proportionnelle à un quotient de la valeur de différence et d'une quantité de gaz respiratoire inspiratoire (S30) fournie au patient avec la participation du dispositif de ventilation (10) pendant la course respiratoire antérieure.

**6.** Procédé selon la revendication 4 ou 5,
**caractérisé en ce que** la détermination de la valeur initiale du contenu de tractus respiratoire en fonction de la valeur de décision comprend une comparaison de la valeur de décision avec une valeur de seuil de décision prédéterminée, la valeur initiale du contenu de tractus respiratoire étant déterminée en fonction d'un résultat de la comparaison (S30).

**7.** Procédé selon l'une quelconque des revendications précédentes, incluant la revendication 3,
**caractérisé en ce que** la valeur initiale de réinitialisation est déterminée comme étant la valeur initiale du contenu de tractus respiratoire, indépendamment de la différence de quantité de gaz respiratoire, si, auparavant, lors d'un premier nombre prédéterminé de déterminations successives de la valeur initiale du contenu de tractus respiratoire, la valeur initiale de continuité a été déterminée à chaque fois comme étant la valeur initiale du contenu de tractus respiratoire (S40).

**8.** Procédé selon l'une quelconque des revendications précédentes, incluant la revendication 3,
**caractérisé en ce que** la valeur initiale de réinitialisation est déterminée comme étant la valeur initiale du contenu de tractus respiratoire, indépendamment de la différence de quantité de gaz respiratoire, si, auparavant, au cours d'un deuxième nombre prédéterminé de déterminations de la valeur initiale de contenu respiratoire, le nombre de déterminations ayant abouti à la valeur initiale de continuité atteint ou dépasse un seuil de proportion prédéterminé, le seuil de proportion étant inférieur au deuxième nombre.

**9.** Procédé selon l'une quelconque des revendications précédentes, incluant la revendication 3,
**caractérisé en ce que** la valeur initiale de réinitialisation est déterminée indépendamment de la différence de quantité de gaz respiratoire en tant que valeur initiale de contenu de tractus respiratoire si, auparavant, dans un nombre prédéterminé de courses respiratoires, la fréquence à laquelle un quotient d'une première différence de contenu de tractus respiratoire et d'une deuxième différence de contenu de tractus respiratoire différente de la première atteint ou dépasse une valeur seuil de quotient prédéterminée atteint ou dépasse une valeur seuil de fréquence, de préférence, la première différence de contenu du tractus respiratoire étant une différence entre le contenu du tractus respiratoire en gaz respiratoire à la fin d'une course respiratoire déterminée et le contenu du tractus respiratoire en gaz respiratoire à la fin d'une course respiratoire antérieure précédant la course respiratoire déterminée ou/et la deuxième différence de contenu du tractus respiratoire étant une différence entre un contenu du tractus respiratoire en gaz respiratoire à la fin d'une phase inspiratoire de la course respiratoire déterminée et le contenu du tractus respiratoire en gaz respiratoire à la fin de la course respiratoire antérieure précédant la course respiratoire déterminée (S50).

**10.** Procédé selon l'une quelconque des revendications précédentes,
**caractérisé en ce que** la valeur initiale de réinitialisation est déterminée indépendamment de la différence de quantité de gaz respiratoire comme étant la valeur initiale du contenu respiratoire si, pour au moins l'une des deux courses respiratoires subséquentes actuelles pour lesquelles la valeur initiale du contenu respiratoire est déterminée, il est déterminé qu'un volume de gaz respiratoire expiratoire de la course respiratoire antérieure, déterminé à partir des flux de gaz respiratoire expiratoire détectés, est supérieur en valeur absolue à un volume de gaz respiratoire inspiratoire de la même course respiratoire antérieure, déterminé à partir des flux de gaz respiratoire inspiratoire.

**11.** Procédé selon l'une des revendications précédentes,
**caractérisé en ce que** la valeur initiale de réinitialisation est déterminée indépendamment de la différence de quantité de gaz respiratoire comme étant la valeur initiale de contenu de tractus respiratoire si un changement quantitatif de la PEP a été détecté (S70) pendant une période de temps prédéterminée avant la détermination actuelle de la valeur initiale de contenu de tractus respiratoire.

12. Procédé selon l'une des revendications précédentes,
**caractérisé en ce que** le contenu de tractus respiratoire en gaz respiratoire est délivré graphiquement en fonction du temps au niveau d'un dispositif de sortie (28), dans lequel, en particulier, lorsque la valeur initiale de continuité est définie comme étant la valeur initiale de contenu de tractus respiratoire, la sortie graphique du contenu de tractus respiratoire en fonction du temps est modifiée.

13. Dispositif de ventilation (10) pour la ventilation au moins partiellement artificielle d'un patient (12), comprenant :

- un dispositif de source de gaz respiratoire (15) qui fournit un gaz respiratoire inspiratoire pour la ventilation artificielle du patient (12),
- un dispositif de modification de l'écoulement (16) qui est conçu pour générer et modifier la quantité d'un flux de gaz respiratoire inspiratoire (AF),
- un ensemble de conduits de gaz respiratoire (30) ayant une extrémité longitudinale proximale plus proche du patient (12) en fonctionnement et une extrémité longitudinale distale plus éloignée du patient (12) en fonctionnement, pour transporter le flux de gaz respiratoire inspiratoire (AF) de l'ensemble de sources de gaz respiratoire (15) vers le patient (12),
- un ensemble de capteurs de flux (44) qui est conçu pour détecter la valeur du flux de gaz respiratoire inspiratoire (AF) ainsi qu'un flux de gaz respiratoire expiratoire (EF),
- un dispositif de commande (18) avec une mémoire de données (19), le dispositif de commande (18) étant relié par transmission de signaux à la mémoire de données (19) et au dispositif de détection de flux (44) et étant conçu pour commander la puissance de fonctionnement du dispositif de modification de flux (16) pour modifier le flux de gaz respiratoire inspiratoire (AF),

**caractérisé en ce que** le dispositif de commande (16) est adapté pour mettre en œuvre le procédé selon l'une quelconque des revendications précédentes, de préférence le dispositif respiratoire (10) comprenant un dispositif de sortie graphique (28) et le dispositif de commande (28) étant adapté pour mettre en œuvre le procédé selon la revendication 12.

14. Dispositif de ventilation (10) selon la revendication 13,
**caractérisé en ce que** le dispositif de ventilation (10) est adapté pour effectuer une ventilation artificielle dans un mode de ventilation assistée dans lequel le dispositif de commande (16) détecte un effort inspiratoire du patient ventilé (12) et, en réponse à la détection de l'effort inspiratoire, commande le dispositif de modification de l'écoulement (16) pour administrer une quantité de gaz respiratoire inspiratoire au patient (12) par l'intermédiaire de l'ensemble de conduits de gaz respiratoire (30), de préférence, le dispositif de commande (18) étant conçu pour détecter une expiration incomplète sur la base de la valeur initiale déterminée du contenu de tractus respiratoire ou/et sur la base du contenu déterminé de tractus respiratoire en gaz respiratoire, et ensuite, lorsque le dispositif de commande (18) a détecté une expiration incomplète, pour modifier le mode de ventilation, en particulier pour passer d'un mode de ventilation commandé en fonction du volume à un mode de ventilation commandé en fonction de la pression.

15. Dispositif de ventilation (10) selon l'une des revendications 13 à 14,
**caractérisé en ce que** le dispositif de détection de flux (44), sur la base des valeurs de détection duquel la valeur initiale du contenu de tractus respiratoire est déterminée, est un dispositif de détection de flux proximal (44).

**Fig. 1**

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019094736 A1
- US 8757152 B1